# EUROPEAN PATENT APPLICATION

(11) **EP 2 251 346 A2**
(43) Date of publication of application: **17.11.2010**
(21) Application number: 10168887.7
(22) Date of filing: 24.04.2004
(51) Int. Cl.: C07J 31/00

(54) **Process for preparing guggulsterones and guggulsterol**

(30) Priority: 24.04.2003 KR 20030025904; 24.04.2003 KR 20030025919; 24.04.2003 KR 20030025936; 24.04.2003 KR 20030025962; 24.04.2003 KR 20030025989
(62) Divisional of application: 04729374.1
(71) Applicant: Seoul National University Industry Foundation, Seoul 151-818 (KR)
(72) Inventor: Kang, Heonjoong, Seongnam-city Kyeongki-do 463-749 (KR); Ham, Jungyeob, Seoul 151-770 (KR); Chin, Jungwook, Yeongdeungpo-gu Seoul 150-096 (KR)
(74) Representative: Albrecht, Thomas

(57) **Abstract**

The present invention relates to a method for selective preparing 4,17 (20)-*E*-pregnadiene-3,16-dione (*E*-guggulsterone) of the formula (III) and 4,17 (20)-*Z*-pregnadiene-3,16-dione (*Z*-guggulsterone) of the formula (IV) having an effect of lowering the elevated low density lipoprotein (LDL) and high levels of the cholesterol effectively, and elevating the low levels of the high density lipoprotein (HDL) from a easy-available steroid of the following formula (I).

Also, the present invention provides a method for preparation of the compound of the above formula (II).

## Description

### FIELD OF THE INVENTION

The present invention relates to a method for preparing 4,17(20)-pregnadiene-3,16-dione (hereinafter, referred to "guggulsterone") having an effect of lowering the elevated low density lipoprotein (LDL) and high levels of the cholesterol effectively, and elevating the low levels of the high density lipoprotein (HDL), a method for preparation of the Z-type isomer of the guggulsterone from the *E*-type, and a method for preparing the compound of the formula (II), the intermediate of the above the compound (III).

### BACKGROUND OF THE INVENTION

The guggulsterone has been extracted from the Indian guggul tree (botanical name; *Commiphora mukul*), and used traditionally for curing hyperlipidemia, and also is known as an effective antagonist for the famesoid orphan receptor (FXR), which regulates the cholesterol metabolism *(*Nature, 2002, June, 411.; Science, 2002, May, 1703). Conventionally, the *E*-type or Z-type of the guggulsterone has been used as a hypolipidemic drug, and it has been obtained by extracting the resin of the guggul tree, and, if necessary, further purifying it by using HPLC, etc. A method of synthesis of guggulsterone mixture was disclosed in the EP 0 447 706 A1. As shown in the following scheme, the ketone and acetate groups of 16-dehydropregnalone acetate (1) was reduced with lithium aluminum hydride (LiAlH₄) to give 5,16-pregnadiene- 3β ,20 diol (2), which was reacted with acetic anhydride and *p*-toluenesulfonic acid to afford the mixture of 5,17(20)-*trans*-pregnadien-3β,16β-diacetate and 5,17(20)-*trans*-pregnadien-3β,16α-diacetate (3). The resultant mixture was reacted with potassium hydroxide in methanol to give the mixture of 5,17(20)-*trans*-pregnadien-3β,16β-diol and 5,17(20)-*trans-*pregnadien-3β,16α-diol (4), which was oxidized with aluminum isobutoxide or aluminum phenoxide to thereby yield the stereoisomeric mixture (Z: E= about 8:2) of guggulsterones.

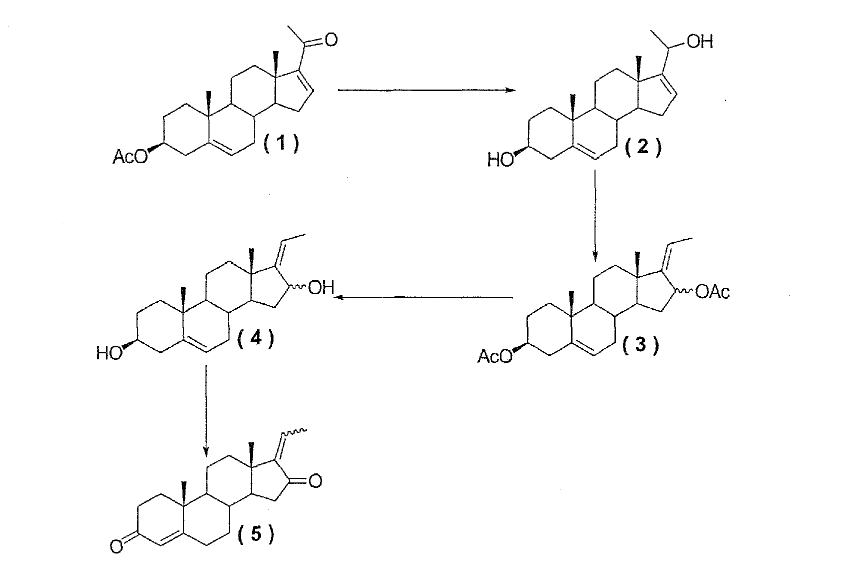

Although the above compound (5) of the method in EP 0 447 706 A1 has known to have a good efficacy, the manufacturing method thereof is not satisfactory, thereby being not cost-effective. That is to say,
1) The starting material, 16-dehydropregnalone is not a commonly available steroid, and so it is not proper to be applied to the industry.
2) When lithium aluminum hydride (LiAlH₄) used at the first reduction step is exposed to the moisture, hydrogen gas is generated, which may lead to a possibility of explosion, thereby being very dangerous to adopt it in an industrial scale.
3) It is difficult to dispose the acetic acid being used as a solvent in the second reaction after completion of the reaction, and also the reaction time of 72 hours is too long.
4) At the third reaction step, the reaction time to reflux methanol with potassium hydroxide for 6 hours is comparatively long.
5) The yield of the third reaction step appears 114%, but the yield of the final product, guggulsterone is not described on the patent specification.
6) The method of the synthesis described in the above patent is not reliable, since the analysis data of guggulsterone and the intermediate thereof are not described on the patent specification.

According to the method disclosed in J. Org. Chem., 1964, 29, 1142*,* 16α,17α-epoxypregnenolone was reacted with hydrazine in 85% purity at 190°C for 5.5 hours to synthesize diol intermediate compound, which was a mixture of *trans* and *cis.* Since the yield of the diol compound was 21.7%, it is impossible to prepare the final product, guggulsterone in an industrial scale.

Moreover, the method disclosed in J. Org. Chem., 1964, 29, 1142 has problems as follows.
1) Since the yield of the resultant diol compound(8a) is 21.7%, it is not proper to be applied to the industry.
2) The resultant diol compound(8a) is obtained as a mixture, which has no optical activity, so that it is necessary to isolate it additionally. And, the final product has low efficacy as medicine or food.

Under the circumstance, a novel method for preparation of single *E type* or *Z type* guggulsterone with easiness and low cost has been demanded in the art.

### SUMMARY OF THE INVENTION

The object of the present invention is to provide an economical method for preparation of guggulsterol, single *E*-type guggulsterone (III), and single *Z*-type guggulsterone (IV) with a high yield, and in a short reaction time from a starting material, which is available easily at a low price.

Under the foregoing circumstance, the present inventors carried out an extensive investigation on the synthesis of guggulsterone. So, they found that the diol compound in 91% yield could be prepared selectively by reacting 16, 17-epoxypregnenolone with hydrazine in 95% purity at 160°C within 2 hours. As a result, they found efficient methods for preparation of the compound represented by the following formula (II) from a known steroid represented by the following formula (I), and syntheses of the guggulsterones represented by the following formula (III) and (IV) easily and economically.

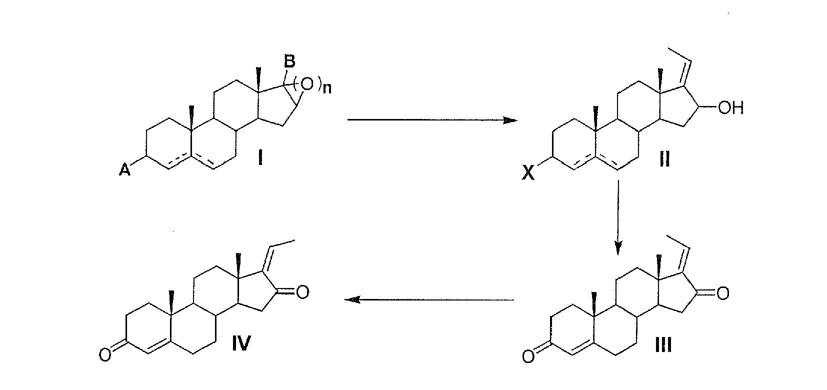

Wherein A represents a hydroxyl (-OH) group or an oxo (=O) group; B represents a hydroxyl (-OH) group, an oxo (=O) group or a methyl ketone (-C(O)CH₃) group; X represents a hydroxyl (-OH) group or an oxo (=O) group; n is a number of oxygen atom, 0 or 1; and a dotted line indicates the presence of a double bond at the position C-4 (hereinafter, referred to "Δ^{4"}) or C-5 (hereinafter, referred to "Δ⁵").

### DESCRIPTION OF THE PREFERRED EMBODIMENT

The compound of the formula (III), an object of the present invention, can be obtained by oxidizing the compound of the formula (II) and the compound of the formula (IV) can be obtained by isomerizing the compound of the formula (III). The above method of preparation will be explained, after explaining a method for preparation of the compound of the formula (II) from the compound of the formula (I) as a starting material.

### I. Synthesis of the compound of the formula (II):

### I-1. Synthesis of the compound of the formula (II-A) from 4-androsten-3,17-dione (I-A) of the formula (I) wherein A and B respectively represent an oxo (=O) group , n is 0, and Δ⁴:

The compound of the formula (I-A) was treated with 1,2-ethanedithiol for protecting the ketone group only at the position C-3 to give the compound of the formula (I-A-1), which was subjected to Wittig reaction on the ketone group at the position C-17 to give the compound of the formula (I-A-2). The compound of the formula (I-A-3) was obtained by eliminating the protecting group of ketone group at the position C-3 of the above compound of the formula (I-A-2). The compound of the formula (I-A-3) was oxidized by reacting with selenium dioxide (SeO₂), in order to introduce the alcohol group at the position C-16, thereby yielding the guggulsterol represented by the compound of the formula (II-A).

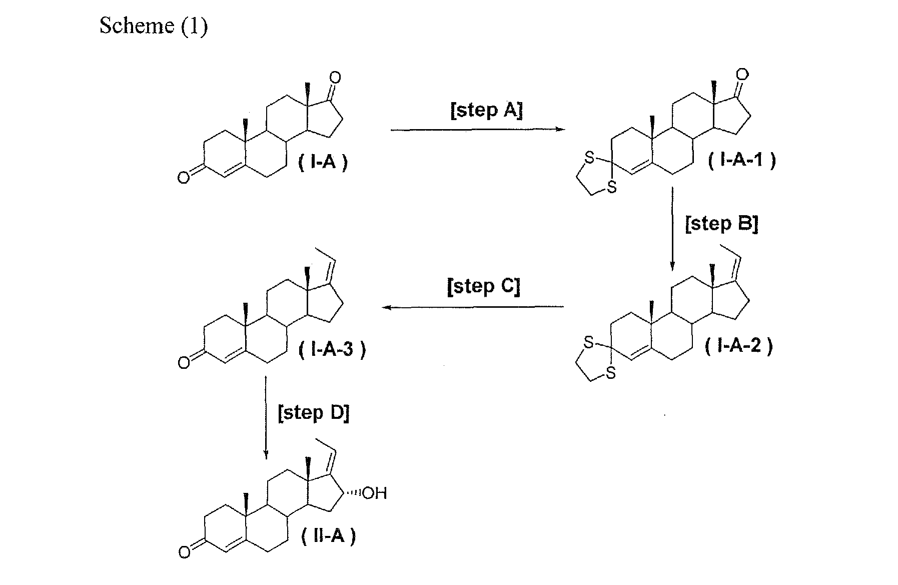

### [Step A] Preparation of the compound of the formula (I-A-1):

The compound of the formula (I-A-1) can be obtained by reacting the compound of the formula (I-A) with alkanedithiol in a solvent in the presence of an acid catalyst.

As the solvents usable in this reaction, dichloromethane, chloroform, 1,2-dichloroethane, diethyl ether, acetonitrile, tetrahydrofuran, toluene, and glacial acetic acid can be given. These solvents may be used either singly or a combination of two or more. Among them, dichloromethane and glacial acetic acid are particularly preferred.

As the alkanedithiol which protects selectively a conjugated at the position C-3, 1,2-ethanedithiol and 1,3-propanedithiol can be given, with a preference of 1,2-ethanedithiol.

As the Lewis acids, boron trifluoro etherate (BF₃ ·Et₂O), zinc iodide (ZnI₂), tellium chloride (TeCl₂), magnesium iodide (MgI₂) and *p*-toluenesulfonic acid (TsOH) can be given. Among them, BF₃ ·Et₂O and *p*-toluenesulfonic acid are particularly preferred. The amount of Lewis acid used in this reaction is generally 0.1 to 2.0 equivalent to the amount of alkanedithiol, with a preference of 0.5 to 1.0 equivalent.

The reaction temperature depends on the solvent used. However, it is preferred to conduct the reaction at -10 to 80 °C, more preferably at 0 to 25 °C. The reaction time also depends on the reaction temperature and the solvent used. However, it is preferred to conduct the reaction for 30 minutes to 12 hours, more preferably for less than 4 hours.

### [Step B] Preparation of the compound of the formula (I-A-2).

In order to prepare the compound of the formula (I-A-2), it is preferred to react the compound of the formula (I-A-1) with ethyltriphenyl phosphonium halide (Ph₃P⁺CH₂CH₃X⁻) in a non-aqueous solvent in the presence of a strong base.

As the non-aqueous solvents, diethylether, tetrahydrofuran, hexane, heptane, methanol, ethanol, dimethyl sulfoxide, benzene, toluene, and xylene can be given. These solvents may be used either singly or a combination of two or more. Among them, diethylether and tetrahydrofuran are particularly preferred.

As the ethyltriphenyl phosphonium halide used in the Wittig reaction, ethyltriphenyl phosphonium chloride (Ph₃P⁺CH₂CH₃Cl⁻), ethyltriphenyl phosphonium bromide (Ph₃P⁺CH₂CH₃Br⁻) and ethyltriphenyl phosphonium iodide (Ph₃P⁺CH₂CH₃I⁻) can be given. Among them, ethyltriphenyl phosphonium bromide and ethyltriphenyl phosphonium iodide are particularly preferred.

As the strong base reagent used in the Wittig reaction, it includes lithium hydride, sodium hydride, potassium hydride, n-butyl lithium, sec-butyl lithium, tert-butyl lithium, sodium methoxide, sodium ethoxide, or potassium t-butoxide. Among them, n-butyl lithium and potassium t-butoxide are particularly preferred.

The amount of a base used is generally 1.0 to 4.0 equivalents to that of ethyltriphenyl phosphonium halide for forming ylide, with a preference of 1.0 to 1.5 equivalents.

The reaction temperature varies as the type of solvent used, yet it generally lies at -10 to 120°C. When ethyltriphenyl phosphonium halide is reacted with the strong base in the presence of anhydrous solvent in order to form ylide, the reaction is carried out at -10 to 20°C, and when the compound of the formula (I-A-1) is used for the Wittig reaction, the reaction is carried out at -10°C to the boiling point of the solvent used, with a preference at -10 to 20°C when the solvent being tetrahydrofuran.

The reaction time also depends on the reaction temperature and the solvent used. However, it is preferred to conduct the reaction for 30 minutes to 1 day, more preferably for less than 2 hours.

### [Step C] Preparation of the compound of the formula (I-A-3).

The compound of the formula (I-A-3) can be obtained by eliminating the protecting group of ketone group at the position C-3 of the compound of the formula (I-A-2).

The suitable solvents in the deprotecting reaction include distilled water, acetone, chloroform, dichloromethane, acetonitrile, tetrahydrofuran, and glacial acetic acid. These solvents may be used either singly or in a combination of two or more. Among them, tetrahydrofuran and glacial acetic acid are particularly preferred.

As the reagents used in the deprotecting reaction, silver nitrate (AgNO₂), *N*-bromosuccinimide (NBS), iodine, and selenium dioxide (SeO₂) can be given. These reagents may be used either singly or in a combination of two or more. Among them, silver nitrate with iodine, and selenium dioxide are particularly preferred.

The amount of the deprotecting reagent depends on the reagent used. However, it is preferred to use 0.5 to 3.0 equivalents to the amount of the compound of the formula (I-A-2), with a preference of 0.5 to 1.5 equivalents.

The reaction temperature depends on the solvent and reagent used. However, it is preferred to conduct the reaction at -10 to 100°C, more preferably at 10 to 65°C. The reaction time also depends on the reaction temperature and the type of reagent used, yet it generally lies between 30 minutes and 1 day, preferably for less than 3 hours.

### [Step D] Preparation of guggulsterol of the formula (II-A).

Guggulsterol of the formula (II-A) can be obtained by reacting the compound of the formula (I-A-3) with selenium oxides (SeO₂) and a hydrogen peroxide in a solvent.

The amount of selenium dioxide is generally 0.1 to 2.0 equivalents to that of the compound of the formula (I-A-3), with a preference of 0.25 to 1.0 equivalent.

As the peroxides, hydrogen peroxide, t-butyl peroxide, *N*-methylmorpholine *N*-oxide (NMO), or pyridine N-oxide (C₅H₅N-O) can be given. Among them, t-butyl peroxide is particularly preferred. The amount of hydrogen peroxide is generally 0.5 to 3.0 equivalents to that of the compound of the formula (I-A-3), preferably 1.0 to 2.0 equivalents.

As the aprotic polar solvents usable in this process, *N*,*N*-dimethylformamide, *N*,*N*-dimethylacetamide, dimethylsulfoxide, pyridine, acetonitrile, acetone, ethylacetate, tetrachloroethane, chloroform, and dichloromethane can be given. As the ethers, tetrahydrofuran, dioxane, dimethoxyethane, diethylene glycol dimethylether, and triethylene glycol dimethylether can be given. As the aromatic hydrocarbons, benzene, toluene, and xylene can be given. Among them, aprotic polar solvents and ethers are preferred. More specifically, dichloromethane and dioxane are preferred.

The reaction temperature depends on the solvent used. However, it is preferred to conduct the reaction at -10 to 80°C, more preferably at 10 to 30°C. The reaction time also varies according to the reaction temperature and the type of solvent used, yet the reaction is generally conducted for 30 minutes to 12 hours, preferably for less than 2 hours.

### I-2. Synthesis of the compound of the formula (II-B) from 5-androsten-3-ol-17-one [DHEA (I-B)] of the formula (I) wherein A represents a hydroxy (-OH) group, B is an oxo (=O) group, n is 0, and Δ⁵:

The compound of the formula (I-B-1) can be obtained by Wittig reaction on the ketone group at the position C-17 of the compound of the formula (I-B), without protecting the alcohol group at the position C-3, as shown in the following scheme 2. The compound of the formula (II-B) can be obtained by reacting the compound of the formula (I-B-1) with selenium dioxide (SeO₂), thereby introducing the alcohol group at the position C-16.

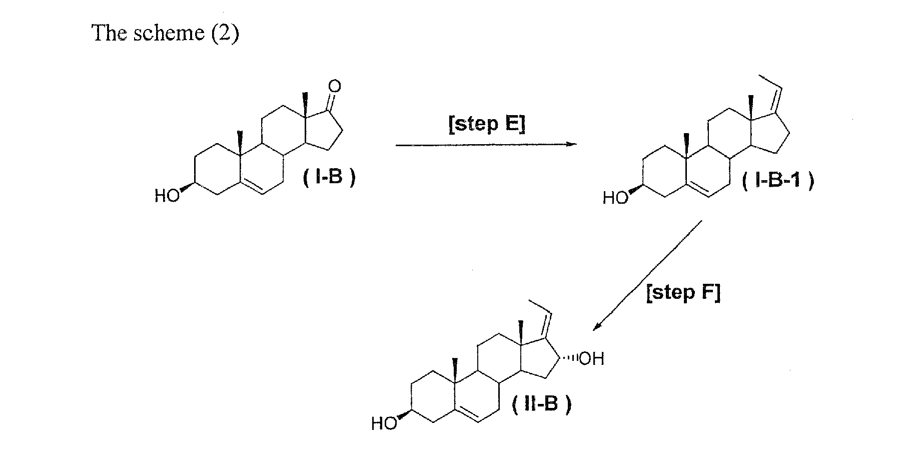

### [Step E] Preparation of the compound of the formula (I-B-1).

In order to obtain the compound of the formula (I-B-1), it is preferred to react the compound of the formula (I-B) with ethyltriphenyl phosphonium halide (Ph₃P⁺CH₂CH₃X⁻) in a non-aqueous solvent in the presence of a strong base, without protecting the alcohol group at the position C-3.

As the non-aqueous solvents, diethylether, tetrahydrofuran, dimethyl sulfoxide, benzene, toluene, and xylene can be given. These solvents may be used either singly or in a combination of two or more. Among them, tetrahydrofuran and dimethyl sulfoxide are particularly preferred.

As the ethyltriphenyl phosphonium halides used in the Wittig reaction, ethyltriphenyl phosphonium chloride (Ph₃P⁺CH₂CH₃Cl⁻), ethyltriphenyl phosphonium bromide (Ph₃P⁺CH_{Z}CH₃Br⁻) and ethyltriphenyl phosphonium iodide (Ph₃P⁺CH₂CH³I⁻) can be given. Among them, ethyltriphenyl phosphonium bromide and ethyltriphenyl phosphonium are particularly preferred.

As the strong base reagents, lithium hydride, sodium hydride, potassium hydride, n-butyl lithium, sec-butyl lithium, tert-butyl lithium, sodium ethoxide, and potassium t-butoxide can be given. Among them, sodium hydride, n-butyl lithium, and potassium t-butoxide are particularly preferred.

The amount of a base used is generally 1.0 to 4.0 equivalents to that of ethyltriphenyl phosphonium bromide forming ylide, with a preference of 1.0 to 1.2 equivalents.

The reaction temperature depends on the type of solvent used, yet it is generally preferred to conduct the reaction at -10 to 120°C. When ethyltriphenyl phosphonium bromide reacts to form ylide in the presence of a strong base in non-aqueous solvent, it is preferred to conduct the reaction at -10 to 20 °C, and when the compound of the formula (I-B) is subjected to Wittig reaction, the reaction is carried out at -10°C to the boiling point of the solvent used. Preferably, it is carried out at 25 to 40°C in case of tetrahydrofuran as a solvent; and at 25 to 80°C when dimethyl sulfoxide being used.

The reaction time also varies as the reaction temperature and the type of solvent used, yet it is generallylies between 30 minutes and 1 day, preferably less than 2 hours.

### [Step F] Preparation of the compound of the formula (II-B).

The compound of the formula (II-B) can be obtained by reacting the compound of the formula (I-B-1) with selenium oxides (SeO₂) and a hydrogen peroxide in a solvent.

The amount of selenium dioxide (SeO₂) is generally 0.1 to 3.0 equivalents to that of the compound of the formula (I-B-1), with a preference of 0.5 to 1.5 equivalents.

As the peroxides, hydrogen peroxide, t-butyl peroxide, *N*-methylmorpholine *N*-oxide and pyridine N-oxide can be given. Among them, t-butyl peroxide is particularly preferred. The amount of hydrogen peroxide is generally 0.5 to 4.0 equivalents to that of the compound of the formula (I-B-1), preferably 1.0 to 2.0 equivalents.

As the aprotic polar solvent used in this reaction, *N*,*N*-dimethylformamide, *N*,*N*-dimethylacetamide, dimethylsulfoxide, pyridine, acetonitrile, acetone, ethylacetate, tetrachloroethane, chloroform and dichloromethane can be given. As the ethers, tetrahydrofuran, dioxane, dimethoxyethane, diethylene glycol dimethylether and triethylene glycol dimethylether can be given. As the aromatic hydrocarbon, benzene, toluene and xylene can be given. Among them, aprotic polar solvents and ethers are preferred. More specifically, dichloromethane and dioxane are preferred.

The reaction temperature varies as the type of solvent used, yet the reaction generally proceeds at -10 to 80°C, preferably at -10 to 10°C. The reaction time also varies as the reaction temperature and the type of solvent used, yet the reaction is generally conducted for 30 minutes to 12 hours, preferably for less than 4 hours.

### I-3. Synthesis of the compound of the formula (I-A) from 5-androsten-3-ol-17-one [DHEA (I-B)] of the formula (I) wherein A represents a hydroxy group (-OH), B is an oxo (=O) group, n is 0, and Δ⁵:

The compound of the formula (I-B-1) was obtained by carrying out Wittig reaction on the ketone group at the position C-17 of the compound of the formula (I-B), without protecting the alcohol group at the position C-3, and then it was oxidized with pyridinium chlorochromate (PCC) to form the compound of the formula (I-B-2) with the ketone group at the position C-3. The double bond of the compound of the formula (I-B-2) was shifted in the presence of a base to give the compound of the formula (I-B-3), which is, subsequently, reacted with selenium dioxide to introduce the alcohol group at the position C-16, thereby giving the compound of the formula (II-A), guggulsterol.

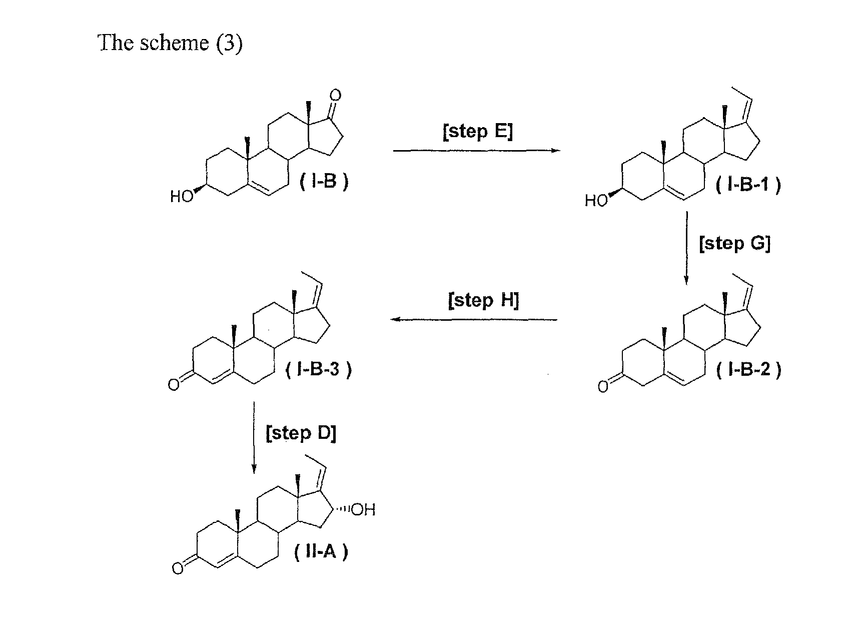

### [Step G] Preparation of the compound of the formula (I-B-2).

The compound of the formula (I-B-2) can be obtained by reacting the compound of the formula (I-B-1) with an oxidizing reagent.

In this oxidation process, pyridinium chlorochromate (PCC), pyridinium dichromate (PDC), Jones' reagent and Swern oxidation reagent (by DMSO+CICOCOCI) can be used for oxidizing a secondary alcohol group into the ketone group at the position C-3. Among them, pyridinium chlorochromate is preferred.

The solvent used varies as the type of an oxidation reagent. Dichloromethane with pyridinium chlorochromate (PCC), *N*,*N*-dimethylacetamide with pyridinium dichromate (PDC), acetone with Jones' reagent, and dichloromethane with Swem oxidation reagent are preferred.

The amount of oxidizing reagent varies as the type of an oxidation reagent. The amount of the chromic oxidizing reagent is generally 1.0 to 5.0 equivalents to that of the compound of the formula (I-B-1), preferably 1.2 to 2.0 equivalents.

In the Swem oxidation reaction, the amount of dimethyl sulfoxide used for making an oxidation reagent is preferably 1.0 to 3.0 equivalents to that of the compound of the formular (I-B-1) and the amount of oxalyl chloride is preferably 1.0 to 1.5 equivalents to that of the compound of the formular (I-B-1).

The reaction temperature varies as the type of an oxidizing reagent, yet it generally lies at -78 to 30°C. It is preferred to conduct the reaction at -10 to 10°C when the chromic oxidizing reagent is used, and at -78 to 30°C when the Swem oxidation reaction is adopted.

The reaction time also varies as the process of oxidation reaction, yet it is generally preferred to conduct the reaction for 30 minutes to 12 hours, more preferably for less than 2 hours.

### [Step H] Preparation of the compound of the formula (I-B-3).

The compound of the formula (I-B-3) can be obtained from the compound of the formula (I-B-2) through a reaction of double bond migration by using a base.

As the solvent usable in this reaction, methanol, ethanol, diethylether, tetrahydrofuran, dimethyl sulfoxide, benzene and toluene can be given. Methanol and tetrahydrofuran are particularly preferred.

As the base used in the elimination reaction of α-hydrogen of ketone group, sodium hydroxide, potassium hydroxide, lithium hydroxide, lithium hydride, sodium hydride, potassium hydride, n-butyl lithium, sec-butyl lithium, tert-butyl lithium, sodium methoxide, sodium ethoxide, and potassium t-butoxide can be given. Among them, sodium hydroxide, potassium hydroxide, and potassium t-butoxide are particularly preferred. The amount of a base is generally 1.0 to 4.0 equivalents to that of the compound of the formula (I-B-2), preferably 1.0 to 1.5 equivalents.

The reaction temperature varies as the type of base and solvent used, yet it is generally preferred to conduct the reaction at -10 to 120°C, more preferably at 10 to 50 °C .

The reaction time varies as reaction temperature and the type of base and solvent used, yet it generally lies between 30 minutes and 4 hours, preferably less than 1 hour.

### I-4. Synthesis of the compound of the formula (II-A) from testosterone (I-C) of the formula (I) wherein A is an oxo (=O) group, B is a hydroxy (-OH) group, n is 0, and Δ⁴:

In the scheme 4, the compound of the formula (I-C) was reacted with 1,2-ethanediol in the presence of an acid catalyst to give the compound of the formula (I-C-1). This compound was subjected to Wittig reaction with ethyltriphenyl phosphonium halide in the presence of a strong base to give the compound of the formula (I-C-3). Subsequently, the compound of the formula (I-A-3) was obtained by deprotecting the ketone group of the compound of the formula (I-C-3), and then it was oxidized by selenium dioxide to introduce the alcohol group at the position C-16 to form the compound of the formula (II-A), guggulsterol

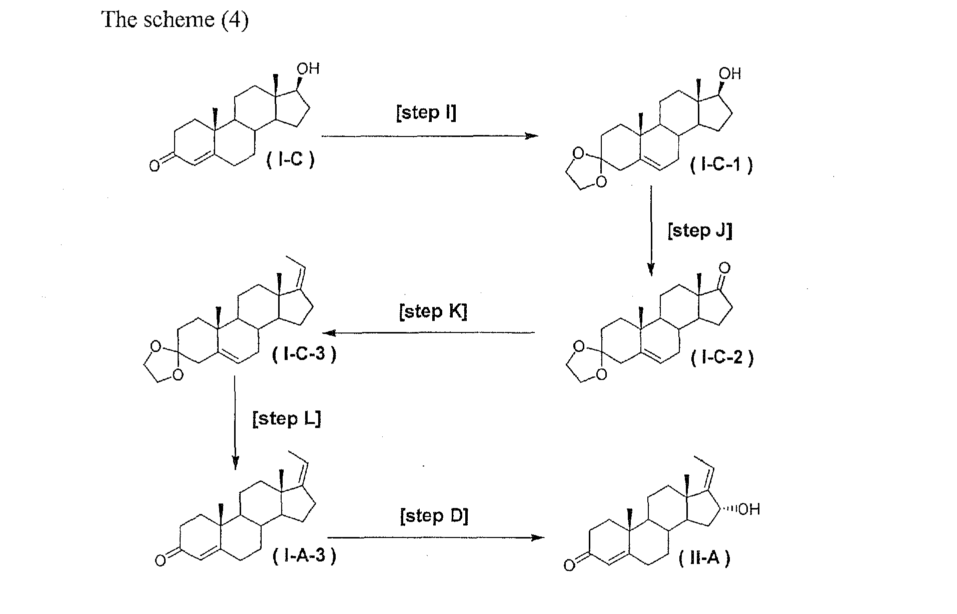

### [Step I] Preparation of the compound of the formula (I-C-1).

In order to prepare the compound of the formula (I-C-1), the compound of the formula (I-C) was reacted with 1,2-ethanediol in the presence of an acid catalyst in a solvent.

As the solvents, chloroform, dichloromethane, tetrachloroethane, tetrahydrofuran, acetonitrile, benzene and toluene can be given. Among them, dichloromethane and benzene solvent are particularly preferred.

As the alkanediols, 1,2-ethanediol (ethylene glycol) and 1,3-propanediol can be given. Among them, 1,2-ethanediol is particularly preferred.

The acid catalysts usable in this reaction include boron trifluoro etherate (BF₃·Et₂O), zinc iodide, oxalic acid and *p*-toluenesulfonic acid. Among them, *p*-toluenesulfonic acid is particularly preferred. The amount of the acid used is generally 0.01 to 2.0 equivalents to that of the alkanediol, with a preference of 0.01 to 0.05 equivalents.

The reaction temperature varies as the type of solvent used, yet it is generally preferred to conduct the reaction at -10 to 110°C, more preferably at 70 to 100°C. The reaction time varies as reaction temperature and the type of solvent used, yet the reaction generally proceeds for 30 minutes to 12 hours, preferably for less than 3 hours.

### [Step J] Preparation of the compound of the formula (I-C-2).

The compound of the formula (I-C-2) was obtained by reacting the compound of the formula (I-C-1) with a chromium oxide.

In this oxidation process, pyridinium chlorochromate (PCC) and pyridinium dichromate (PDC) can be used for oxidizing a secondary alcohol group into the ketone group at the position C-17. Among them, pyridinium chlorochromate is preferred.

The solvent used varies as the type of an oxidation reagent. Dichloromethane with pyridinium chlorochromate (PCC), *N*,*N*-dimethylacetamide with pyridinium dichromate (PDC) are preferred.

The amount of oxidizing reagent varies as the kind of the oxidation reagents, but it is generally 1.0 to 5.0 equivalents to that of the compound of the formula (I-C-1), with a preference of 1.5 to 2.0 equivalents.

The reaction temperature varies as the type of the oxidizing reagents, but it is generally at -10 to 30 °C. The reaction time also varies as the kind of the oxidizing reagents, yet it is generally preferred to conduct the reaction for 30 minutes to 12 hours, more preferably for less than 2 hours.

### [Step K] Preparation of the compound of the formula (I-C-3).

In order to prepare the compound of the formula (I-C-3), it is preferred to react the compound of the formula (I-C-2) with ethyltriphenyl phosphonium halide (Ph₃P⁺CH₂CH₃X⁻) in a non-aqueous solvent in the presence of a strong base.

Suitable non-aqueous solvents in this reaction include diethylether, tetrahydrofuran, hexane, heptane, methanol, ethanol, dimethyl sulfoxide, benzene, toluene, xylene and dimethyl sulfoxide. These solvents may be used either singly or in a combination of two or more. Among them, tetrahydrofuran and dimethyl sulfoxide are particularly preferred.

Suitable ethyltriphenyl phosphonium halides in the Wittig reaction include ethyltriphenyl phosphonium chloride (Ph₃P⁺CH₂CH₃Cl⁻), ethyltriphenyl phosphonium bromide (Ph₃P⁺CH₂CH₃Br⁻) or ethyltriphenyl phosphonium iodide (Ph₃P⁺CH₂CH₃I⁻). Among them, ethyltriphenyl phosphonium bromide and ethyltriphenyl phosphonium iodide are particularly preferred.

Suitable strong base reagents in the Wittig reaction include lithium hydride, sodium hydride, potassium hydride, n-butyl lithium, sec-butyl lithium, tert-butyl lithium, sodium methoxide, sodium ethoxide, and potassium t-butoxide. Among them, sodium hydride and potassium t-butoxide are particularly preferred.

The amount of a base is generally 1.0 to 4.0 equivalents to that of ethyltriphenyl phosphonium halide for forming ylide, but it is generally preferred to have 1.0 to 1.5 equivalents of the base to that of the ethyltriphenyl phosphonium halide.

The reaction temperature varies as the kind of solvents used, yet it is generally at -10 to 120. When ethyltriphenyl phosphonium halide reacts to form ylide in the presence of a strong base in a non-aqueous solvent, the reaction is carried out at -10 to 20°C and when the compound of the formula (I-C-2) is subjected to the Wittig reaction, the reaction is carried out at 25 to 100°C. Preferably, it is carried out at 50 to 70°C when the solvent is tetrahydrofuran and at 20 to 80°C when the solvent is dimethyl sulfoxide.

The reaction time varies as the reaction temperature and the type of the solvent used, yet it is generally preferred to conduct the reaction for 30 minutes to 1 day, more preferably for less than 2 hours.

### [Step L] Preparation of the compound of the formula (I-A-3).

The compound of the formula (I-A-3) can be obtained by eliminating the protecting group of ketone group at the position C-3 of the compound of the formula (I-C-3).

Suitable solvents in the deprotecting reaction include distilled water, acetone, chloroform, dichloromethane, acetonitrile, tetrahydrofuran, and glacial acetic acid. These solvents may be used either singly or in a combination of two or more. Among them, the mixed solvent of distilled water and acetone is particularly preferred.

As the reagents used in the deprotecting reaction, pyridinum *p*-toluenesulfonate (PPTS), *p*-toluenesulfonic acid (TsOH), hydrochloric acid, and glacial acetic acid can be given. Among them, pyridinum *p*-toluenesulfonate (PPTS) is particularly preferred.

The amount of reagent varies as the type of reagents used, yet it is generally 0.05 to 0.5 equivalents to that of the compound of the formula (I-C-3), with a preference of 0.1 to 0.3 equivalents.

The reaction temperature varies as the type of solvents and reagents used, yet it is generally at 0 to 100°C, preferably at 30 to 60 °C. The reaction time also varies as the reaction temperature and the type of reagents used, yet it is generally 30 minutes to 1 day, preferably less than 3 hours.

### I-5. Synthesis of the compound of the formula (II-B) from 16α ,17α -epoxypregnenolone (I-D) of the formula (I) wherein A is a hydroxy (-OH) group, and B is a methyl ketone (-C(O)CH₃) group, n is 1, and Δ⁵:

In the scheme 5, the compound of the formula (I-D) was reacted with hydrazine (NH₂NH₂) in the presence of a base at high temperature to synthesize the compound of the formula (II-B) wherein C is a hydroxy (-OH) group and Δ⁵.

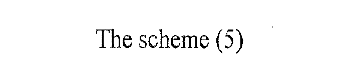

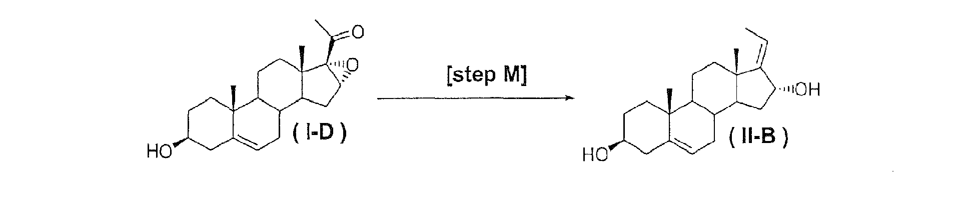

### [Step M] Preparation of the compound of the formula (II-B).

In this process, hydrazine (NH₂NH₂) monohydrate and hydrazine (NH₂NH₂) anhydride can be used. Among them, hydrazine monohydrate in 95∼98% purity is preferred. The amount of hydrazine used is 1.0 to 40 equivalents to that of that of the compound of the formula (I-D), with a preference of 1.5 to 2.5 equivalents to the organometal reagent used as a base, and 10 to 20 equivalents to that of the alkali metal hydroxide used as a base.

Suitable solvents in this reaction include methanol, ethanol, butanol, ethylether, tetrahydrofuran, 1,4-dioxane, dimethyl sulfoxide, benzene, toluene, xylene, diethylene glycol dimethylether, diethylene glycol monomethylether, and diethylene glycol. Among them, tetrahydrofuran and diethylene glycol are particularly preferred.

As the bases used, sodium hydroxide, potassium hydroxide, lithium hydroxide, n-butyl lithium, sec-butyl lithium, tert-butyl lithium, lithium diisopropylamide, potassium diisopropylamide, sodium methoxide, sodium ethoxide, and potassium t-butoxide can be given. Among them, sodium hydroxide, potassium hydroxide and potassium diisopropylamide are preferred. The amount of the base used varies as the type of the base used, but it is preferred to use 2.0 to 15 equivalents with alkali metal hydroxide, 1.0 to 3.0 equivalents with alkali metal hydride, 1.0 to 3.0 equivalents with organometal reagent, 1.0 to 4.0 equivalents with alkali metal alcholate.

The reaction temperature varies as the type of solvent and base used, yet it is generally at -100 to 190, preferably at -100 to -78°C for alkali metal hydride and organometal reagent, at 20 to 100°C for alkali metal alcholate, at 80 to 160 °C for alkali metal hydroxide. The reaction time also varies as the reaction temperature and the type of base used, yet it is generally preferred to conduct the reaction for 30 minutes to 48 hours, more preferably for less than 4 hours.

### I-6. Synthesis of the compound of the formula (II-A) from progesterone of the formula (I-E) wherein A is an oxo (=O) group, B is a methyl ketone (-C(O)CH₃) group, n is 0, and Δ⁴:

In the scheme 6, the ketone groups at the position C-3 and C-20 of the compound of the formula (I-E) were reduced to the alcohols thereof, the compound of the formula (I-E-1), and then, this compound was reacted using an selective oxidizing agent, thereby oxidizing the alcohol group at the position C-3 into a ketone group to give the compound of the formula (I-E-2). After preparing the compound of the formula (I-E-3) by introducing a *p*-toluene sulfonyl group or a methane sulfonyl group as a leaving group at the alcohol group of the position C-20, it was reacted with a base to form the compound of the formula (I-E-4), which was the *E* and *Z* isomeric mixture. The mixture was reacted with selenium dioxide to give guggulsterol, the compound of the formula (II-A), by introducing an alcohol group at the position C-16.

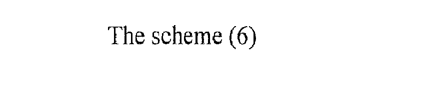

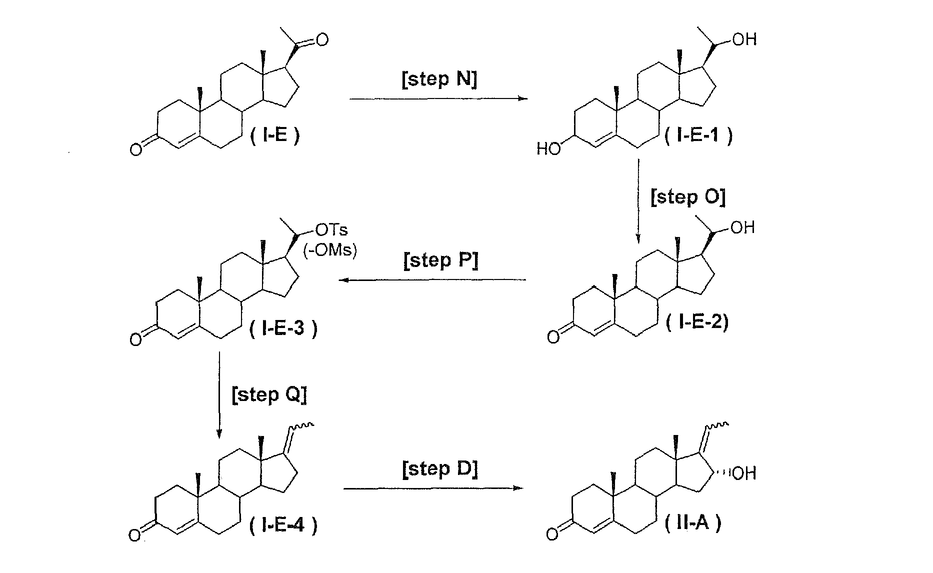

### [Step N] Preparation of the compound of the formula (I-E-1).

The compound of the formula (I-E-1) was obtained by reacting the compound of the formula (I-E) with a reducing reagent.

Suitable solvents in this reaction include methanol, ethanol, diethylether, tetrahydrofuran and dichloromethane. Among them, methanol and ethanol are particularly preferred.

As the reducing agent used in the ketone reduction, lithium aluminum hydride (LAH), diisobutyl aluminum hydride (DIBAL-H), sodium boron hydride (NaBH₄), lithium boron hydride (LiBH₄), and platinum catalyst in the hydride reduction reaction can be given. Among them, boron hydride reducing reagent is preferred and sodium boron hydride (NaBH₄) is particularly preferred. The amount of reducing agent is generally 0.5 to 4.0 equivalents to that of the compound of the formula (I-E), with a preference of 1.5 to 2.5 equivalents.

The reaction temperature varies as the type of solvent and reducing agent used, yet it is generally at -100 to 60°C, preferably at -78 to 25°C. The reaction time varies as the reaction temperature and the type of solvent used, yet it is generally preferred to conduct the reaction for 30 minutes to 6 hours, preferably for less than 2 hours.

### [Step O] Preparation of the compound of the formula (I-E-2).

The compound of the formula (I-E-2) can be obtained by reacting the compound of the formula (I-E-1) with a selective oxidizing reagent.

Activated manganese dioxide (MnO₂) can be used in order to oxidize an alcohol group selectively in this process. The amount of oxidizing reagent is generally 1.0 to 40 equivalents to that of the compound of the formula (I-E-1), with a preference of 10 to 20 equivalents.

Suitable solvents in this reaction include pentane, hexane, heptane, petroleum ether, benzene, toluene, xylene, acetone, chloroform, dichloromethane and tetrachloroethane. Among them, dichloromethane is particularly preferred.

The reaction temperature varies as the kind of solvent used, yet it is generally at -10 to 100°C, preferably at 20 to 50°C. The reaction time varies as the reaction temperature and the kind of solvent used, yet it is generally preferred to conduct the reaction for 30 minutes to 12 hours, more preferably for less than 2 hours.

### [Step P] Preparation of the compound of the formula (I-E-3).

The compound of the formula (I-E-3) can be obtained by reacting the compound of the formula (I-E-2) withp-toluene sulfonyl chloride or methane sulfonyl chloride in the presence of a base.

In this reaction, *p*-toluene sulfonyl chloride or methane sulfonyl chloride can be used. The amount of the regent is generally 1.0 to 4.0 equivalents to that of the compound of the formula (I-E-2), with a preference of 2.0 to 3.0 equivalents.

Suitable solvents in this reaction include *N*,*N*-dimethylformamide, *N*,*N*-dimethylacetamide, dimethyl sulfoxide, acetonitrile, acetone, ethylacetate, tetrahydrofuran, tetrachloroethane, chloroform, dichloromethane, benzene, toluene or pyridine. Among them, dichloromethane and pyridine are preferred.

As the base used, pyridine, triethylamine, imidazole, and *N*,*N*-dimethyl aminopyridine can be given. Among them, pyridine and *N*,*N*-dimethylaminopyridine are preferred. The amount of the base is generally 1.0 to 4.0 equivalents to that of the compound of the formula (I-E-2), with a preference of 1.5 to 2.5 equivalent.

The reaction temperature varies as the type of solvent used, yet it is generally at -10 to 40°C, preferably at 0 to 25°C. The reaction time varies as the reaction temperature and the kind of solvent used, yet it is generally preferred to conduct the reaction for 1 hour to 1 day, more preferably for less than 5 hours.

### [Step Q] Preparation of the compound of the formula (I-E-4).

The compound of the formula (I-E-4) can be obtained by reacting the compound of the formula (I-E-3) with a strong base.

As the base reagent used in this reaction, sodium hydride, potassium hydride, sodium methoxide, sodium ethoxide, potassium t-butoxide, pyridine, 1,5-diazabicyclo[4,3,0]non-5-ene, and the amine salt of 1,8-diazabicyclo[5,4,0]undec-7-ene can be given. Among them, sodium methoxide and pyridine are preferred. The amount of a base is generally 1.0 to 4.0 equivalents to that of an alkaline metal hydride, an alkali metal alcholate and an amine salt, respectively. Pyridine can be used as a solvent and also be used as a reactant.

Suitable solvents in this reaction include *N*,*N*-dimethylformamide, *N*,*N*-dimethylacetamide, dimethyl sulfoxide, acetonitrile, methanol, ethanol, butanol, tetrahydrofuran, tetrachloroethane, chloroform, dichloromethane, benzene, toluene and pyridine. Among them, dimethyl sulfoxide, methanol and pyridine are preferred.

The reaction temperature varies as the type of the solvent and the base used, yet it is generally at -78 to 150°C and is preferably at 25 to 120°C. The reaction time varies as the reaction temperature and the type of the base used, yet the reaction is generally conducted for 1 hour to 1 day, more preferably for less than 5 hours.

### II. Synthesis of the compound of the formula (III):

### II-1. Synthesis of the compound of the formula (III), E-type guggulsterone, from guggulsterol of the formula (II-A):

In the scheme 7, the compound of the formula (III), the *E*-type guggulsterone, can be obtained by reacting the compound of the formula (II-A) with an oxidation reagent.

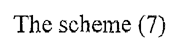

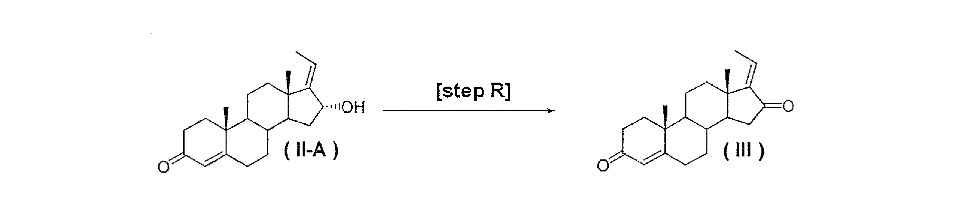

### [Step R] Preparation of the E-type guggulsterone, the compound of the formula (III).

The *E*-type guggulsterone of the formula (III) can be obtained by reacting guggulsterol of the formula (II-A) with an oxidizing agent.

As the oxidizing reagent used for oxidizing a secondary alcohol group into a ketone group, pyridinium chlorochromate (PCC), pyridinium dichromate (PDC), Jones' reagent, and activated manganese dioxide can be given. Among them, Jones' reagent and activated manganese dioxide are preferred. Activated manganese dioxide is particularly preferred.

Suitable solvents in this reaction include pentane, hexane, heptane, petroleum ether, benzene, toluene, xylene, dimethyl sulfoxide, acetone, chloroform, dichloromethane, tetrachloroethane, diethylether and tetrahydrofuran. Among them, dichloromethane is preferred. The activated manganese dioxide can be used in this reaction without solvent.

The amount of the oxidizing reagent varies as the type of an oxidizing reagent used, yet it is generally 1.0 to 20 equivalents to that of the compound of the formula (II-A), with a preference of 5.0 to 10 equivalents to activated manganese dioxide.

The reaction temperature varies as the type of solvent used, yet it is generally at -10 to 100°C, preferably at 20 to 50°C. The reaction time varies as the reaction temperature and the type of solvent used, yet it is generally preferred to conduct the reaction for 30 minutes to 12 hours, more preferably for less than 2 hours.

### II-2. Synthesis of the compound of the formula (III), the E-type guggulsterone, from the compound of the formula (II-B):

In the scheme 8, the compound of the formula (III), the *E*-type guggulsterone, can be obtained by reacting the compound of the formula (II-B) with an oxidizing reagent.

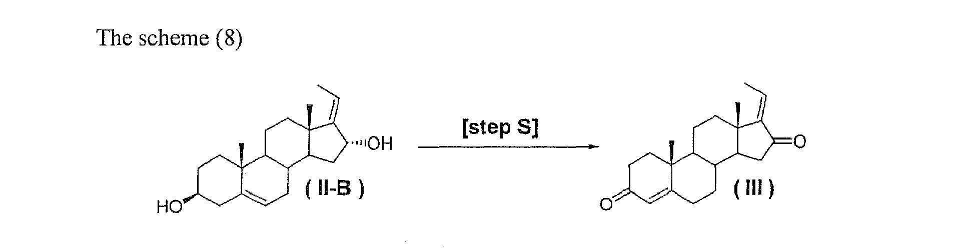

### [Step S] Preparation of E type guggulsterone of the formula (III).

Guggulsterone of the formula (III) can be obtained by Oppenauer oxidation reaction, reacting the compound of the formula (II-B) with an aluminum oxidation reagent.

As the aluminum oxidation reagents used in the Oppenauer oxidation reaction, aluminum isopropoxide (Al[OCH(CH₃)₂]₃), aluminum tri *sec*-butoxide (Al[OCH(CH₃)CH₂CH₃]₃), aluminum tri *tert*-butoxide (Al[OC(CH₃)₃]₃), and aluminum phenoxide (Al[OC₆H₅]₃) can be given. Among them, aluminium isopropoxide is preferred. The amount of an aluminum oxidation reagent is generally 0.3 to 1.5 equivalents to that of the compound of the formula (II-B), with a preference of 0.5 to 1.0 equivalent.

Suitable non-aqueous solvents in this reaction include benzene, toluene, xylene and mesitylene. Among them, benzene and toluene are preferred.

As the reducing reagents usable in the Oppenauer oxidation reaction, acetone, ethylmethylketone, acetophenone, benzophenone and cyclohexanone can be given. Among them, ethylmethylketone and cyclohexanone are preferred.

The reaction temperature varies as the type of solvent used, yet it is generally at 50 to 180°C. The reaction temperature affects the regioselectivity of the final product, guggulsterone. The *E*-type guggulsterone only is obtained when the raction is performed at 50 to 100°C, while the *E*, *Z*-guggulsterone mixture is obtained the reaction being performed at 120 to 180 °C.

The reaction time varies as the reaction temperature and the type of used solvent, yet it is generally preerred to conduct the reaction for 30 minutes to 4 hours, more preferably for less than 2 hours.

### III. Synthesis of the compound of the formula (IV):

### Synthesis of the compound of the formula (IV), the Z-type guggulsterone from the compound of the formula (III), the E-type guggulsterone:

In the scheme 9, the Z-type guggulsterone of the formula (IV) can be obtained by a photochemical reaction, a thermochemical reaction or an acid catalyst reaction of the E-type guggulsterone of the formula (III).

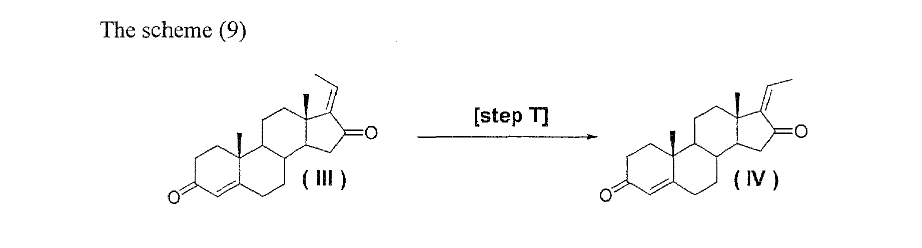

### [Process T] Preparation of the Z-guggulsterone of the formula (IV).

As the photosensitizer in the photochemical reaction of the *E*-type guggulsterone, methylene blue, methylene green and Rose Bengal can be given. The amount of the photosensitizer is generally 0.01 to 0.2 equivalents to that of the compound of the formula (III), with a preference of 0.05 to 0.1 equivalents.

As the source of light, a 60W to 500W tungsten bulb can be generally used, yet it is preferred to use a 150W to 300W tungsten bulb.

Suitable solvents in the photochemical reaction include distilled water, methanol, ethanol, acetonitrile, acetone, tetrachloroethane, chloroform, dichloromethane, tetrahydrofuran, benzene, toluene, and xylene. These solvents may be used either singly or in a combination of two or more. Among them, dichloromethane, chloroform, acetonitrile, methanol and ethanol are preferred. Chloroform and methanol are particularly preferred.

The reaction temperature varies as the type of solvent used, yet it is generally at -10 to 100°C and is preferably at 20 to 50°C.

The reaction time varies as the strength of the source of light and the reaction temperature and the type of photosensitizer used, yet it is generally preferred to conduct the reaction for 1 hour to 48 hours, more preferably for less than 10 hours.

Suitable solvents in the thermochemical reaction include benzene, toluene, xylene and mesitylene. Among them, toluene is preferred.

The reaction temperature varies as the type of used solvent, yet it is generally at 80 to 200°C and is preferably at 100 to 130°C.

The reaction time varies as the reaction temperature and the type of solvent used, yet it is generally preferred to conduct the reaction for 1 hour to 48 hours, more preferably for less than 2 hours.

As the acid catalyst used in the acid catalyst reaction, *p*-toluenesulfonic acid and dilute hydrochloric acid (0.1 to 3.0 N HCl) can be given. The amount of the acid catalyst is 0.01 to 0.5 equivalents to that of the compound of the formula (III), with a preference of 0.05 to 0.2 equivalents.

Suitable solvents in the acid catalyst reaction include acetonitrile, acetone, tetrachloroethane, chloroform, dichloromethane, tetrahydrofuran, benzene, toluene, and xylene. These solvents may be used either singly or in a combination of two or more. Among them, dichloromethane, chloroform, acetonitrile, benzene and toluene are preferred. More preferably, chloroform and benzene are used for *p*-toluenesulfonic acid, and acetonitrile is used for dilute hydrochloric acid, respectively.

The reaction temperature varies as the type of solvent used, yet it is generally at -10 to 160°C, preferably at 30 to 80°C.

The reaction time varies as the reaction temperature and the type of used acid catalyst, yet it is generally preferred to conduct the reaction for 30 minutes to 10 hours, more preferably for less than 1 hour.

The *E*-type guggulsterone, the compound of the formula (III), and the Z-type guggulsterone, the compound of the formula (VI), are known as the medicine for curing human hyperlipidemia.

### EXAMPLES

The present invention is more specifically illustrated by the following examples. However, it should be understood that the present invention is not limited by these examples in any manner.

### Example 1: Synthesis of cyclo-3-(1,2-ethanediyl dithioacetal)-4-androsten-17-one of the formula (I-A-1) from the compound of the formula (I-A) [Step A]

4-Androsten-3,17-dione (I-A) (28.6 g, 100 mmol) was completely dissolved in glacial acetic acid (100 mℓ) at 25°C, and 1,2-ethanedithiol (10.4 mℓ, 110mmol) and *p*-toluenesulfonic acid (8.6 g, 50 mmol) was added to the above solution continuously. The reaction mixture was further reacted at the room temperature, and the resulted mixture was poured into the distilled water (100 mℓ), and then was stirred thoroughly at the room temperature for 15 minutes. The resulted mixture was extracted with ethyl acetate (3 x 50 mℓ), and the organic layer was washed with brine and distilled water, and dried over magnesium sulfate, filtered and evaporated under the reduced pressure. And the resulted residue was purified by column chromatography with ethyl acetate/hexane (2/8) to thereby yield the title compound (27.2 g, yield: 75%).
¹H NMR (300MHz, CDCl₃): 5.51 (s, 1H), 3.37 (m, 3H), 3.23 (m, 1H), 2.39 (q, 1H), 2.17-0.84 (steroid main-body and 1.04 (s, 3H), 0.88 (s, 3H) =24H)
¹³C NMR (75.5MHz, CDCl₃): 221.3, 146.1, 125.0, 66.2, 54.6, 51.4, 48.0, 40.4, 40.0, 38.4, 37.7, 37.1, 36.2, 35.7, 32.2, 31.9, 31.8, 22.2, 21.0, 18.9, 14.1

### Example 2: Synthesis of cyclo-3-(1,2-ethanediyl dithioacetal)-4-androsten-17-one of the formula (I-A-1) from formula (I-A) [Step A]

4-Androsten-3,17-dione (I-A) (28.6 g, 100 mmol) was completely dissolved in absolute methanol (150 mℓ) at 0°C, and to this solution, 1,2-ethanedithiol (15 mℓ, 179 mmol) was added and then, the acid catalyst, BF₃ ·Ether (14 mℓ) was slowly added. The reaction was further subjected at 20°C for 10 hours. After completion of the reaction, the resulted mixture was evaporated under the reduced pressure to remove methanol, and is subjected to treat with dichloromethane (100 mℓ) and 5% NaHCO₃ solution. The organic layer was dried over magnesium sulfate, filtered and evaporated under the reduced pressure to thereby yield the title compound (33.4 g, yield: 92%).

### Example 3: Synthesis of cyclo-3-(1,2-ethanediyl dithioacetal)-4,17 (20)-cis-androstene of the formula (I-A-2) from the formula (I-A-1) [Step B]

Ethyltriphenyl phosphonium bromide (44.6 g, 120 mmol) was dissolved in dry tetrahydrofuran (200 mℓ) at 0°C, 120 mℓ (120 mmol). Potassium t-butoxide in anhydrous tetrahydrofuran (1 mol solution) was slowly added to the above solution over 15 minutes. After completion of addition, the reaction mixture was subjected to react further for 30 minutes at the room temperature, and the compound of the formula (I-A-1) (33.4 g, 92.1 mmol) in tetrahydrofuran (150 mℓ) was added slowly at the room temperature. The reaction mixture is stirred at the room temperature for 2 hours for further reaction. The resulted mixture was evaporated under the reduced pressure, and 250 mℓ of the mixed solvent of hexane and ethyl ester (v/v =10/1) was added to form the byproduct, triphenylphosphinooxide (Ph₃P=O) as the precipitates, which were filtered off. The organic layer was washed with brine and distilled water, dried over magnesium sulfate, and filter under the reduced pressure to thereby yield the title compound (33.1 g, yield: 96%).
¹H-NMR (300MHz, CDCl₃): 5.51 (s, 1H), 5.11 (m, 1H), 3.40 (m, 3H), 3.25 (m, 1H), 2.21-0.77 (steroid main-body and 1.05 (s, 3H), 0.91 (s, 3H) = 28H)
¹³C-NMR (75.5MHz, CDCl₃): 150.5, 146.9, 124.5, 113.8, 66.3, 56.2, 54.5, 44.6, 40.4, 40.0, 38.5, 37.7, 37.4, 37.1, 35.7, 33.0, 32.5, 31.8, 24.8, 21.8, 18.9, 17.2, 13.5

### Example 4 Synthesis of cyclo-3-(1,2-ethanediyl dithioacetal)-4,17 (20)-cis-androstene of the formula (I-A-2) from formula (I-A-1) [Step B]

Ethyltriphenyl phosphonium bromide (37.1 g, 100 mmol) was dissolved in anhydrous dimethyl sulfoxide (150 mℓ) at 15°C, and sodium hydride (2.5 g, 105 mmol) was added slowly thereto. After completion of addition, the reaction mixture was stirred for 30 minutes at the room temperature, and the compound of the formula (I-A-1) (30 g, 82.7 mmol) was added slowly at the room temperature. The reaction mixture was stirred for 2 hours at the room temperature. After completion of reaction, brine (200 mℓ) and ethyl acetate (200 mℓ) were poured into the reaction mixture, and the organic layer was separated. The organic layer was dried over magnesium sulfate, filtered and evaporated under the reduced pressure. The mixture of hexane and ethyl ester (v/v = 10/1) (250 mℓ) was added into the resulted residue to form a byproduct, triphenylphosphinooxide (Ph₃P=O) as the precipitate, which was filtered off. The solvent was removed under the reduced pressure to thereby yield the title compound (28.2 g, yield: 91%).

### Example 5 Synthesis of 4,17 (20)-cis-pregnadien-3-one of the formula (I-A-3) from the compound of the formula (I-A-2) [Step C]

The compound of the formula (I-A-2) (33 g, 88 mmol) was dissolved in glacial acetic acid (200 mℓ) at the room temperature, and selenium dioxide (4.8 g, 44mmol) was added to the above mixture at once. The resulted reaction mixture was stirred at the room temperature for 2 hours, and then the distilled water (300 mℓ) was added therein slowly.

The reaction mixture was extracted with ethyl acetate (200 mℓ) three times, and the combined organic layer was washed with 10% NaHCO₃ aqueous solution. The organic layer was dried over magnesium sulfate, filtered, and evaporated under the reduced pressure to dryness. The residue was chromatographed on silica eluting with ethyl acetate/hexane (v/v = 3/7) to thereby yield the title compound (21.8 g, yield: 83%).
¹H NMR (300MHz, CDCl₃): 5.74 (s, 1H), 5.14 (q, 1H), 2.44-0.8 (steroid main-body and 1.71 (d, 3H), 1.23 (s, 3H), 0.93 (s, 3H) = 28H)
¹³C NMR (75.5MHz, CDCl₃): 199.9, 171.7, 150.0, 124.21, 114.1, 56.0, 54.2, 44.5, 39.0, 37.2, 36.1, 35.5, 34.4, 33.3, 32.3, 31.7, 24.8, 21.6, 17.7, 17.2, 13.5

### Example 6 Synthesis of 4,17 (20)-cis-pregnadien-3-one of the formula (I-A-3) from the compound of the formula (I-A-2) [Step C]

Silver nitrate (4.7 g, 30 mmol) and iodide 3.8 g (15 mmol) was added into the 5% tetrahydrofuran in the distilled water (200 mℓ) at the room temperature, followed by stirring for 1 hour. The compound of the formula (I-A-2) (30 g, 80 mmol) was added slowly into the above reaction mixture at the room temperature, followed by stirring for 3 hours at the room temperature. Aqueous saturated sodium thiosulfide solution was added at 0°C, and was extracted with ethyl acetate. The organic layer was dried over magnesium sulfate, filtered, and evaporated under the reduced pressure. The obtained residue was purified by column chromatography with ethyl acetate/hexane (v/v = 3/7) to thereby yield the title compound (21 g, yield: 88%).

### Example 7: Synthesis of E-guggulsterol [4,17 (20)-E-pregnadien-3-one-16α -ol] of the formula (II-A) from the compound of the formula (I-A-3) [Step D]

While selenium dioxide (1.9 g, 18 mmol) was mixed thoroughly in dichloromethane (100 mℓ) at the room temperature, t-butyl peroxide (18 mℓ) was slowly added into the said mixture, followed by stirring at the room temperature for 1 hours. The reaction mixture was cooled to 0 °C, the compound of the formula (I-A-3) (21 g, 70 mmol) in dichloromethane (100 mℓ) was added over 10 minutes. The mixture was heated up to the room temperature, and stirred for 2 hours. After completion of the reaction, the solvent was removed by evaporation under the reduced pressure, and was extracted with ethyl acetate and distilled water. The organic layer was dried over magnesium sulfate, filtered, and evaporated under the reduced pressure to thereby yield the title compound (20 g, yield: 91 %).
¹H-NMR (300MHz, CDCl₃): 5.75 (s, 1H), 5.61 (m, 1H), 4.46 (s, 1H), 2.44-0.94 (steroid main-body and 1.75 (d, 3H), 1.22 (s, 3H), 0.94 (s, 3H) = 27H)
¹³C-NMR (75.5MHz, CDCl₃): 199.8, 171.4, 155.25, 124.3, 120.3, 74.6, 54.1, 52.3, 44.6, 39.0, 37.4, 36.0, 35.4, 34.9, 34.3, 33.2, 21.5, 17.8, 17.7, 13.6

### Example 8: Synthesis of E-guggulsterol [4,17 (20)-E-pregnadien-3-one-16α - ol] of the formula (II-A) from the compound of the formula (I-A-3) [Step D]

While selenium dioxide (3.9 g, 35 mmol) was mixed in dichloromethane (100 mℓ), t-butyl peroxide (18 mℓ) was added by droplet, followed by stirring at the room temperature for 1 hour. The reaction mixture was cooled to 0 °C, and the compound of the formula (I-A-3) (21 g, 70 mmol) in dichloromethane (100 mℓ) was added slowly over 10 minutes. The reaction mixture was warmed up to the room temperature, followed by stirring for 1 hour. After the reaction was completed, the solvent was removed by evaporation under the reduced pressure, extracted with ethyl acetate and distilled water. The combined organic layer was dried over magnesium sulfate, filtered, and evaporated under the reduced pressure to thereby yield the title compound (21 g, yield: 93%).

### Example 9: Synthesis of E-guggulsterol [4,17 (20)-E-pregnadien-3-one-16α - ol] of the formula (II-A) from the compound of the formula (I-A-3) [Step D]

While selenium dioxide (2 g, 18 mmol) was mixed in dioxane (150 mℓ) at 0°C, *N*-methylmorpholine-*N*-oxide (6 g, 50 mmol) was added slowly, followed by stirring for 1 hour. The compound of the formula (I-A-3) (9 g, 35 mmol) was added slowly, the reaction was warmed up to the room temperature, and was stirred for 2 hours. After the reaction was completed, the reaction was evaporated to dryness under the reduced pressure, and the resulting mixture was dissolved in ethyl acetate (100 mℓ). The obtained mixture was washed with brine and distilled water. The organic layer was dried over magnesium sulfate, filtered and evaporated under the reduced pressure to dryness. The residue was purified by column chromatography with ethyl acetate/hexane (v/v = 1/1) to thereby yield the title compound (22 g, yield: 82%).

### Example 10: Synthesis of 5,17 (20)-cis-pregnadien-3β -ol of the formula (I-B-1) from the compound of the formula (I-B) [Step E]

Ethyltriphenyl phosphonium bromide (130 g, 350 mmol) was dissolved in dry dimethyl sulfoxide (400 mℓ) at 15 °C, and sodium hydride (8.6 g, 360 mmol) was added slowly. After confirming completion of addition, the reaction mixture was stirred for an additional 1 hour, and DHEA of the formula (I-B) (28.8 g, 100 mmol) was added to the reaction mixture at the room temperature. The reaction was heated up to 80 °C slowly and stirred for an additional 2 hours. After the reaction was completed, the reaction mixture was extracted with brine (800 mℓ) and ethyl acetate (300 mℓ) three times. The organic layer was dried over magnesium sulfate, filtered, and evaporated under the reduced pressure. Hexane and ethyl ester (v/v = 10/1) (400 mℓ) was added to the residue to form the byproduct, triphenylphosphinooxide (Ph₃P=O) as the precipitate, which was filtered off. The filtrate was evaporated under the reduced pressure to thereby yield the title compound (28.6 g, yield: 95%).
¹H-NMR (300MHz, CDCl₃): 5.38 (d, 1H), 5.16 (m, 1H), 3.53 (m, 1H), 2.42-0.92 (steroid main-body and 1.70 (d, 3H), 1.04 (s, 3H), 0.92 (s, 3H) = 29H)
¹³C-NMR (75.5MHz, CDCl₃): 150.6, 141.2, 122.0, 113.9, 72.1, 56.9, 50.6, 44.4, 42.7, 37.6, 37.4, 37.0, 32.1, 32.0, 31.9, 31.8, 24.9, 21.6, 19.8, 17.0, 13.5

### Example 11: Synthesis of 5,17 (20)-cis-pregnadien-3β -ol of the formula (I-B-1) from the compound of the formula (I-B) [Step E]

Ethyl triphenyl phosphonium bromide (92.8 g, 250 mmol) was dissolved in dry dimethyl sulfoxide (300 mℓ) at 15 °C, and sodium hydride (6.3 g, 260 mmol) was added slowly, the reaction mixture was stirred at the room temperature for an additional 1 hour, and DHEA of the formula (I-B) ( 28.8 g, 100 mmol) was added slowly at the room temperature. The reaction was heated up slowly to 80°C, followed by stirring for an additional 2 hours. After the reaction was completed, the reaction mixture was extracted with brine (600 mℓ) and ethyl acetate (200 mℓ) three times. The combined organic layer was dried over magnesium sulfate, filtered and evaporated under the reduced pressure to dryness. The solution of hexane and ethyl ester (v/v = 10/1) (300 mℓ) was added to the resulting mixture to form a byproduct, triphenylphosphinooxide (Ph₃P=O) as the precipitates, which was filtered off. The filtrate was evaporated under the reduced pressure to dryness. The residue was chromatographed on silica eluting with ethyl acetate/hexane (v/v = 3/7) to thereby yield the title compound (25.5 g, yield: 85%).

### Example 12: Synthesis of 5,17 (20)-cis-pregnadien-3β -ol of the formula (I-B-1) from the compound of the formula (I-B) [Step E]

Ethyltriphenyl phosphonium bromide (92.8 g, 250 mmol) was mixed in dry tetrahydrofuran (300 mℓ) at 15°C, and potassium t-butoxide in dry tetrahydrofuran (1 mol) (280 mℓ, 280 mmol) was added slowly over 15 minutes. After completion of addition, the reaction mixture was stirred at the room temperature for an additional 1 hour, and DHEA of the formula (I-B) (18.2 g, 63 mmol) was added slowly at the room temperature. The reaction mixture was heated up to reflux for 6 hours. After the reaction was completed, the solvent was removed under the reduced pressure, the mixed solution of hexane and ethyl ester (v/v = 10/1) (300 mℓ) was added to form the byproduct, triphenylphosphinooxide (Ph₃P=O)as the precipitate, which was filtered off. The filtrate was evaporated under the reduced pressure to dryness. The resulted residue was chromatographed on silica eluting with ethyl acetate/hexane (v/v = 3/7) to thereby yield the title compound (15.7 g, yield: 83%).

### Example 13: Synthesis of 5,17 (20)-E-pregnadiene- 3β ,16α -diol of the formula (II-B) from the compound of the formula (I-B-1) [Step F]

While selenium dioxide (5.3 g, 476 mmol) was mixed thoroughly in dichloromethane (200 mℓ) at 25 °C, t-butyl peroxide (36 mℓ) was added slowly. After 30 minutes, the reaction was cooled to 0°C, the compound of the formula (I-B-1) (28.6 g, 95.2 mmol) in dichloromethane (100 mℓ) was added over 10 minutes, and was stirred for an additional 3.5 hours. After the reaction was completed, the combined organic layer was washed with brine and distilled water, and dried over magnesium sulfate, filtered and evaporated under the reduced pressure to thereby yield the title compound (27.4 g, yield: 91%).
¹H-NMR (300MHz, CDCl₃): 5.60 (q, 1H), 5.38 (s, 1H), 4.46 (s, 1H), 3.55 (m, 1H), 2.30-0.91 (steroid main-body and 1.86 (d, 3H), 1.05 (s, 3H), 0.91 (s, 3H) = 28H)
¹³C-NMR (75.5MHz, CDCl₃): 155.8, 141.2, 121.9, 120.0, 74.8, 72.1, 53.2, 50.6, 44.6, 42.7, 37.7, 37.6, 37.0, 35.6, 32.0, 31.2, 21.5, 19.7, 17.7, 13.6

### Example 14: Synthesis of 5,17 (20)-E-pregnadiene-3β ,16α - diol of the compound of the formula (II-B) from the compound of the formula (I-B-1) [Step F]

While selenium dioxide (9.2 g, 83.2 mmol)was mixed in dichloromethane (250 mℓ) at 25°C, t-butyl peroxide (36 mℓ) was added slowly therein. After 30 minutes, the reaction mixture was cooled to 0°C, and the compound of the formula (I-B-1) (25 g, 83.2 mmol) in dichloromethane (100 mℓ) was slowly added over 10 minutes thereto. The reaction mixture was stirred at the same temperature for an additional 2 hours. After the reaction was completed, the organic layer was washed with brine and distilled water, and dried over magnesium sulfate. The solvent thereof was evaporated under the reduced pressure to thereby yield to thereby yield the title compound (25.8 g, yield: 98%).

### Example 15: Synthesis of 5,17 (20)-cis-pregnadien-3-one of the formula (I-B-2) from the compound of the formula (I-B-1) [Step G]

The compound of the formula (I-B-1, 25 g, 83 mmol) was dissolved in dichloromethane (250 mℓ) at 0°C, and then the molecular sieves 4Å (3 g) and pyridinium chlorochromate (PCC, 21.5 g, 100 mmol) were added. The reaction mixture was stirred at the same temperature for 2 hours, and the reaction was terminated by adding ethylether (250 mℓ). The resultant mixture was preliminary purified by removing the byproduct through passing the flash silica column, and the filtrate was evaporated to dryness. The residue was chromatographed on silica eluting with ethyl acetate/ hexane (v/v = 3/7) to thereby yield the title compound (22.5 g, yield: 91 %).
¹H-NMR (300MHz, CDCl₃): 5.38 (t, 1H), 5.17 (m, 1H), 3.28 (d, 1H), 2.85 (q, 1H), 2.82-0.80 (steroid main-body and 1.70 (d, 3H), 1.22 (s, 3H), 0.94 (s, 3H) = 26H)
¹³C-NMR (75.5MHz, CDCl₃): 210.6, 150.3, 139.0, 123.2, 114.0, 56.8, 49.6, 48.7, 44.5, 38.0, 37.4, 37.3, 37.2, 32.0, 31.9, 31.8, 24.9, 21.9, 19.6, 17.1, 13.5

### Example 16: Synthesis of 5,17 (20)-cis-pregnadien-3-one of the formula (I-B-2) from the compound of the formula (I-B-1) [Step G]

The compound of the formula (I-B-1) (5.0 g, 17 mmol) was dissolved in acetone (200 mℓ) at 10°C, and then 8N Jones' reagent (3 mℓ) was added slowly. The reaction was maintained at the same temperature for 30 minutes, and the solvent was evaporated under the reduced pressure. Subsequently, the obtained mixture was extracted with ethyl acetate and brine. The organic layer was dried over magnesium sulfate, filtered, and evaporated under the reduced pressure. The resultant mixture was purified by column chromatography (eluent: ethyl acetate/ hexane solvent= 3/7) to thereby yield the title compound (3.8 g, yield: 74%).

### Example 17: Synthesis of 4,17 (20)-cis-pregnadien-3-one of the formula (I-B-3) from the compound of the formula (I-B-2) [Step H]

The compound of the formula (I-B-2, 22 g, 73.7 mmol) was dissolved in methanol (100 mℓ) at the room temperature, and then potassium hydroxide (5 g) was dissolved therein. The reaction mixture was heated up to reflux for 40 minutes, and then was cooled down to the room temperature. The solvent was removed by evaporation under the reduced pressure and the mixture was extracted with ethyl acetate and brine. The organic layer was dried over magnesium sulfate, filtered, and evaporated under the reduced pressure to thereby yield the title compound (20 g, yield: 91 %).
¹H-NMR (300MHz, CDCl₃): 5.74 (s, 1H), 5.14 (q, 1H), 2.44-0.8 (steroid main-body and 1.71 (d, 3H), 1.23 (s, 3H), 0.93 (s, 3H) = 28H)
¹³C NMR (75.5MHz, CDCl₃): 199.9, 171.7, 150.0, 124.21, 114.1, 56.0, 54.2, 44.5, 39.0, 37.2, 36.1, 35.5, 34.4, 33.3, 32.3, 31.7, 24.8, 21.6, 17.7, 17.2, 13.5

### Example 18: Synthesis of 4,17 (20)-cis-pregnadien-3-one of the formula (I-B-3) from the compound of the formula (I-B-2) [Step H]

The compound of the formula (I-B-2, 15 g, 50 mmol) was dissolved in dry tetrahydrofuran (80 mℓ) at 10°C, and then potassium t-butoxide in dry tetrahydrofuran (1 M solution, 60 mℓ, 60 mmol) was added slowly for 15 minutes. The reaction was heated to the room temperature slowly, followed by stirring for an additional 1 hour. Subsequently, the solvent was evaporation under the reduced pressure to dryness, and the resultant mixture was extracted with ethyl acetate and brine. The obtained organic layer was dried over magnesium sulfate, filtered, and evaporated under the reduced pressure to thereby yield the title compound (14.2 g, yield: 95%).

### Example 19: Synthesis of cyclo-3-(1,2-ethanediyl acetal)-5-androsten-17β -ol of the formula (I-C-1) from the compound of the formula (I-C) [Step I]

Testosterone (I-C, 28.8 g, 100 mmol) was dissolved in benzene (200 mℓ ) at 25 °C, and 1,2-ethanediol (6.2 mℓ, 110mmol) and *p*-toluenesulfonic acid (0.2 g, 1 mmol) were added therein. The mixture was heated up to reflux in a Dean-Stark apparatus to remove the moisture for 3 hours. After the reaction was completed, the reaction mixture was cooled down to the room temperature, and the saturated NaHCO₃ solution (100 mℓ) was added thereto to separate the benzene layer, and the remaining aqueous layer was extracted with ethyl acetate (80 mℓ) three times. The combined organic layer was dried over magnesium sulfate, filtered and evaporated under the reduced pressure. The residue was purified by silica gel column chromatography (eluent: ethyl acetate/hexane= 3/7) to thereby yield the title compound (25.3 g, yield: 76%).
¹H NMR (300MHz, CDCl₃): 5.35 (t, 1H), 3.97 (q, 4H), 3.66 (t, 1H), 2.56 (q, 1H), 2.10-0.77 (steroid main-body and 1.06 (s, 3H), 0.78 (s, 3H) = 25H)
¹³C NMR (75.5MHz, CDCl₃): 140.7, 122.2, 109.8, 82.2, 64.8, 64.6, 51.8, 50.3, 43.2, 42.2, 37.1, 37.0, 36.7, 32.4, 31.8, 31.5, 31.0, 23.8, 21.1, 19.3, 11.3

### Example 20: Synthesis of cyclo-3-(1,2-ethanediyl acetal)-5-androsten-17β -ol of the formula (I-C-1) from the compound of the formula (I-C) [Step I]

Testosterone (I-C, 28.8 g, 100 mmol) was dissolved in benzene (200 mℓ) at 25°C and 1,2-ethanediol (18.6 mℓ, 330 mmol) and p-toluenesulfonic acid (1 g, 5 mmol) were added therein. The mixture was heated up to reflux in a Dean-Stark apparatus to remove the moisture for 3 hours. After completion of the reaction, the reaction mixture was cooled to the room temperature, and the saturated NaHCO₃ solution (100 mℓ) was added thereto to separate the benzene layer, and the remaining aqueous layer was extracted with ethyl acetate (80 mℓ) three times. The combined organic layer was dried over magnesium sulfate, filtered and evaporated under the reduced pressure. The residue was purified by silica gel column chromatography (eluent: ethyl acetate/hexane= 3/7) to thereby yield the title compound (29 g, yield: 87%).

### Example 21: Synthesis of cyclo-3-(1,2-ethanediyl dithioacetal)-5-androsten-17-one of the formula (I-C-2) from the compound of the formula (I-C-1) [Step J]

The compound of the formula (I-C-1, 28 g, 84 mmol) was dissolved in dichloromethane (250 mℓ) at 0°C, and then molecular sieves 4Å (3 g) and pyridinium chlorochromate (PCC, 21.5 g, 100 mmol) were added therein. The reaction mixture was stirred at the same temperature for an additional 2 hours, and the reaction was terminated by adding ethylether (200 mℓ). The reaction mixture was subjected to the flash column to remove the byproduct, and the solvent was removed by evaporation. And the residue was purified by silica gel column chromatography (eluent: ethyl acetate/hexane= 3/7) to thereby yield the title compound (23 g, yield: 83%).
¹H-NMR (300MHz, CDCl₃): 5.29 (s, 1H), 3.87 (s, 1H), 2.48 (d, 1H), 2.26 (g, 1H), 2.04-0.98 (steroid main-body and 0.98 (s, 3H), 0.81 (s, 3H) = 26H)
¹³C-NMR (75.5MHz, CDCl₃): 221.2, 140.7, 121.7, 109.6, 64.8, 64.6, 52.0, 50.1, 47.8, 42.1, 37.1, 36.6, 36.1, 31.8, 31.8, 31.3, 31.0, 30.1, 22.2, 20.7,19.2, 13.9

### Example 22: Synthesis of cyclo-3-(1,2-ethanediyl dithioacetal)-5-androsten-17-one of the formula (I-C-2) from the compound of the formula (I-C-1) [Step J]

The compound of the formula (I-C-1, 28 g, 84 mmol) was dissolved in dichloromethane (300 mℓ) at 0°C, and then the molecular sieves 4Å (5 g) and pyridinium chlorochromate (36 g, 168 mmol) were added therein. The reaction mixture was stirred at the same temperature for an additional 2 hours, and the reaction was terminated by adding ethylether (250 mℓ). The reaction mixture was subjected to the molecular sieves to remove the byproduct, and the solvent was removed by evaporation. And the residue was purified by silica gel column chromatography (eluent: ethyl acetate/hexane= 3/7) to thereby yield the title compound (24.4 g, yield: 88%).

### Example 23: Synthesis of cyclo-3-(1,2-ethanediyl acetal)-5,17 (20)-cis-androstene of the formula (I-C-3) from the compound of the formula (I-C-2) [Step K]

Ethyltriphenyl phosphonium bromide (37 g, 100 mmol) was dissolved in anhydrous tetrahydrofuran (200 mℓ) at 0°C, and then potassium t-butoxide in anhydrous tetrahydrofuran (1 mol solution) (110 mℓ, 110 mmol) was added slowly for 15 minutes. After completion of addition, the reaction mixture was reacted at the room temperature for an additional 30 minutes, the compound of the formula (I-C-2, 24 g, 83.2 mmol) in tetrahydrofuran (150 mℓ) was added slowly at the room temperature. The reaction mixture was heated to reflux for 2 hours. After the reaction was completed, the reaction was cooled to the room temperature. The reaction mixture was evaporated under the reduced pressure to dryness, and the residue was poured into the mixed solution of hexane and ethyl ester (v/v = 10/1, 250 mℓ) to precipitate triphenyl phosphate oxide (Ph₃P=O), which was filtered off. The organic layer was washed with brine and distilled water, dried over magnesium sulfate, filtered and evaporated under the reduced pressure. The residue was purified by silica gel column chromatography (eluent: ethyl acetate/hexane= 1/9) to thereby yield the title compound (23.1 g, yield: 81%).
¹H-NMR (300MHz, CDCl₃): 5.38 (t, 1H), 5.16 (q, 1H), 3.97 (m, 4H), 2.57 (q, 1H), 2.23-0.89 (steroid main-body and 1.68 (d, 3H), 1.06 (s, 3H), 0.92 (s, 3H) = 27H)
¹³C-NMR (75.5MHz, CDCl₃): 150.6, 140.5, 122.5, 113.9, 109.9, 64.9, 64.6, 56.9, 50.1, 44.4, 42.2, 37.4, 37.1, 36.7, 31.8, 31.8, 31.5, 24.9, 21.6, 19.2, 17.0, 13.5

### Example 24: Synthesis of cyclo-3-(1,2-ethanediyl acetal)-5,17 (20)-cis-androstene of the formula (I-C-3) from the compound of the formula (I-C-2) [Step K]

Ethyltriphenyl phosphonium bromide (40 g, 105 mmol) was dissolved in anhydrous tetrahydrofuran (250 mℓ) at 10°C, and then sodium hydride (2.6 g, 110 mmol) was added slowly. After confirming completion of addition, the reaction mixture was stirred at the room temperature for an additional 30 minutes, and the compound of the formula (I-C-2, 24 g, 83.2 mmol) was added slowly at the room temperature. The reaction mixture was stirred at the room temperature for 2 hours. After the reaction was completed, the reaction mixture was washed with brine (200 mℓ) and ethyl acetate (200 mℓ), and the organic layer was separated. The organic layer was dried over magnesium sulfate, filtered and evaporated under the reduced pressure. The residue was poured into the mixed solution of hexane and ethyl ester (v/v = 10/1, 250 mℓ) to precipitate triphenyl phosphate oxide (Ph₃P=O), which was filtered off. The filtrate was evaporated under the reduced pressure to thereby yield the title compound (26.5 g, yield: 93%).

### Example 25: Synthesis of 4,17 (20)-cis-pregnadien-3-one of the formula (I-A-3) from the compound of the formula (I-C-3) [Step L]

The compound of the formula (I-C-3, 26 g, 76 mmol) was dissolved in 5% distilled water in acetone (200 mℓ), and then pyridinum *p*-toluenesulfonate(PPTS, 1.8 g, 7 mmol) was added therein. The reaction mixture was heated to 40 °C for 2 hours. After the reaction was completed, acetone was removed under the reduced pressure, and the reaction mixture was extracted with distilled water (100 mℓ) and ethyl acetate (150 mℓ). The organic layer was dried over magnesium sulfate, filtered, and evaporated under the reduced pressure to thereby yield the title compound (21.3 g, yield: 94%).
¹H-NMR (300MHz, CDCl₃): 5.74 (s, 1H), 5.14 (q, 1H), 2.44-0.8 (m, 19H), 1.71 (d, 3H), 1.23 (s, 3H), 0.93 (s, 3H)
¹³C NMR (75.5MHz, CDCl₃): 199.9, 171.7, 150.0, 124.21, 114.1, 56.0, 54.2, 44.5, 39.0, 37.2, 36.1, 35.5, 34.4, 33.3, 32.3, 31.7, 24.8, 21.6, 17.7, 17.2, 13.5

### Example 26: Synthesis of 4,17 (20)-cis-pregnadien-3-one of the formula (I-A-3) from the compound of the formula (I-C-3) [Step L]

The compound of the formula (I-C-3, 5 g, 15 mmol) was mixed with 75% glacial acetic acid (45 mℓ), and then the mixture was heated to 60 °C for 30 minutes. After the reaction was completed, the reaction was cooled to the room temperature, and the reaction mixture was extracted with brine (30 mℓ) and ethyl acetate (50 mℓ x 3). Remaining glacial acetic acid in the organic layer was removed by adding the saturated aqueous NaHCO₃ solution and distilled water, the organic layer was dried over magnesium sulfate, filtered, and the solvent was evaporated under the reduced pressure to dryness. The resulted residue was purified by the column chromatography (eluent: ethyl acetate/hexane= 3/7) to thereby yield the title compound (3.8 g, yield: 84%).

### Example 27: Synthesis of 5,17 (20)-E-pregnadiene-3β ,16α - diol of the formula (II-B) from the compound of the formula (I-D) [Step M]

The compound of the formula (I-D, 33.1 g, 100 mmol) and potassium hydroxide (50 g) were dissolved in diethylene glycol (200 mℓ) at the room temperature, and then hydrazine monohydrate (purity : 98%, 97 mℓ, 2 mol) was added therein. The reaction mixture was heated up to 120°C to reflux for 1 hours, and after the condenser was removed, the reaction was proceeded at 160°C for an additional 2 hours. After confirming the completion of the reaction by TLC, the reaction was cooled to the room temperature. The reaction mixture was mixed with distilled water (200 mℓ) and then extracted with chloroform (200 mℓ) three times. The organic layer was dried over magnesium sulfate, filtered, and the solvent was removed by evaporation under the reduced pressure to thereby yield the title compound (28.8 g, yield: 91%).
¹H-NMR (300MHz, CDCl₃): 5.60 (q, 1H), 5.38 (s, 1H), 4.46 (s, 1H), 3.55 (m, 1H), 2.30-0.91 (steroid main-body and 1.86 (d, 3H), 1.05 (s, 3H), 0.91 (s, 3H) = 28H)
¹³C-NMR (75.5MHz, CDCl₃): 155.8, 141.2, 121.9, 120.0, 74.8, 72.1, 53.2, 50.6, 44.6, 42.7, 37.7, 37.6, 37.0, 35.6, 32.0, 32.0, 31.2,21.5, 19.7, 17.7, 13.6

### Example 28: Synthesis of 4-pregnene-3,20-diol of the formula (I-E-1) from the compound of the formula (I-E) [Step N]

Progesterone of the formula (I-E, 31.5 g, 100 mmol) was dissolved in methanol (300 mℓ) at 0°C, and then sodium borohydride (NaBH₄, 7.6 g, 200 mmol) was carefully added therein. The reaction was proceeded at 20 °C for 2 hours, and the solvent was removed by evaporation under the reduced pressure. The reaction mixture was extracted with ethyl acetate and brine, and the organic layer was dried over magnesium sulfate, filtered and evaporated under the reduced pressure to thereby yield the title compound (31.2 g, yield: 98%).
¹H-NMR (300MHz, CDCl₃): 5.30 (s, 1H), 4.16 (s, 1H), 3.74 (d, 1H), 2.22-0.77 (steroid main-body and 1.15 (d, 3H), 1.08 (s, 3H), 0.80 (s, 3H) = 29H)
¹³C NMR (75.5MHz, CDCl₃): 148.0, 123.8, 70.9, 68.3, 59.0, 56.1, 54.9, 42.8, 40.4, 37.8, 36.3, 35.8, 33.6, 32.6, 29.9, 26.0, 24.9, 24.0, 21.3, 19.3, 12.8

### Example 29: Synthesis of 4-pregnene-3,20-diol of the formula (I-E-1) from the compound of the formula (I-E) [Step N]

Progesterone of the formula (I-E, 31.5 g, 100 mmol) was dissolved in anhydrous dichloromethane (300 mℓ) at -78 °C, and then 1.0 M diisobutyl aluminum hydride (DIBAL-H) in hexane (220 mℓ) was carefully added therein. After the reaction was proceeded at the same temperature for 2 hours, the reaction was heated to -20°C, and ethyl acetate (5 mℓ) was added, followed by stirring for an additional 20 minutes. The reaction was warmed up to 0°C, and then the excess redution reagent was removed by adding the distilled water (1 mℓ) slowly. The organic layer was extracted with 10% sulfuric acid, washed with distilled water again, and dried over magnesium sulfate. It was filtered and evaporated under the reduced pressure to thereby yield the title compound (30.6 g, yield: 96%).

### Example 30: Synthesis of 20-hydroxy-4-preguen-3-one of the formula (I-E-2) from the compound of the formula (I-E-1) [Step O]

The compound of the formula (I-E-1, 30 g, 94 mmol) was dissolved in dichloromethane (300 mℓ) at the room temperature, and then activated manganese oxide (81 g, 940 mmol) was added at once. The reaction was heated to 40°C, followed by stirring for 2 hours vigorously. After the reaction was completed, the reaction mixture was passed through a flash silica column to remove manganese oxide. The solvent was removed by evaporation under the reduced pressure to thereby yield the title compound (27.4 g, yield: 92%).
¹H-NMR (300MHz, CDCl₃): 5.74 (s, 1H), 3.74 (d, 1H), 2.40-0.79 (steroid main-body and 1.20 (s, 3H), 1.15 (d, 3H), 0.82 (s, 3H) = 30H)
¹³C NMR (75.5MHz, CDCl₃): 200.0, 171.8, 124.2, 70.9, 58.8, 55.8, 54.2, 42.8, 40.1, 39.0, 36.1, 35.9, 34.4, 33.3, 32.5, 26.0, 24.8, 24.1, 21.3, 17.8, 12.8

### Example 31: Synthesis of 20-hydroxy-4-pregnen-3-one of the formula (I-E-2) from the compound of the formula (I-E-1) [Step O]

The compound of the formula (I-E-1, 30 g, 94 mmol) was dissolved in dichloromethane (300 mℓ) at the room temperature, and then activated manganese oxide (163 g, 1.88 mol) was added at once. The reaction was heated to 40°C, followed by stirring for 1 hour vigorously. After the reaction was completed, the reaction mixture was passed through a flash silica column to remove manganese oxide. The solvent was removed by evaporation under the reduced pressure to thereby yield the title compound (28.6 g, yield: 96%).

### Example 32: Synthesis of 20-(p-toluenesulfonyl)-4- prognen-3-one of the formula (I-E-3) from the compound of the formula (I-E-2) [Step P]

The compound of the formula (I-E-2, 28 g, 88.5 mmol) and *N,N-*dimethylaminopyridine (DMAP, 21.6 g, 177 mmol) was dissolved in anhydrous dichloromethane (350 mℓ) at 0°C, and then *p*-toluenesulfonyl chloride (TsCl, 25.3 g, 133 mmol) was added slowly. The reaction was warmed up to the room temperature, followed by stirring for 4 hours. After comfirming the completetion of the reaction by TLC, the organic layer was extracted with aqueous ammonium chloride solution (150 mℓ). The organic layer was dried over magnesium sulfate, filtered and evaporated under the reduced pressure to thereby yield the title compound (35.8 g, yield: 86%).
¹H-NMR (300MHz, CDCl₃): 7.79 (d, 2H), 7.32 (d, 2H), 5.74 (s, 1H), 4.15 (d, 1H), 2.45-0.80 (steroid main-body and 1.15 (s, 3H), 0.83 (s, 3H) = 32H)
¹³C NMR (75.5MHz, CDCl₃): 200.0, 171.2, 144.7, 130.1, 130.1, 127.9, 126.9, 124.2, 82.6, 56.0, 55.7, 54.1, 42.6, 39.0, 38.6, 36.1, 35.9, 34.4, 33.2, 32.4, 25.8, 24.4, 22.0, 21.9, 21.2, 20.9, 17.8, 12.2

### Example 33: Synthesis of 20-(metanesulfonyl)- 4-pregnen-3-one of the formula (I-E-3) from the compound of the formula (I-E-2) [Step P]

The compound of the formula (I-E-2, 28 g, 88.5 mmol) and triethylamine (TEA) (17 mℓ, 124 mmol) were dissolved in anhydrous dichloromethane (250 mℓ) at 0°C, and methane sulfonyl chloride (MsCl, 8.2 mℓ, 106 mmol) was added slowly therein. The reaction mixture was warmed up to the room temperature, and the reaction was maintained for 4 hours. After the reaction was completed, the organic layer extracted with aqueous ammonium chloride (NH₄Cl) solution (100 mℓ). The organic layer was dried over magnesium sulfate, filtered, and the solvent was evaporated under the reduced pressure to thereby yield the title compound (33.2 g, yield: 95%).
¹H-NMR (300MHz, CDCl₃): 5.74 (s, 1H), 4.12 (d, 1H), 3.07 (s, 3H), 2.45-0.80 (steroid main-body and 1.18 (s, 3H), 1.03 (d, 3H), 0.95 (s, 3H) = 29H)
¹³C NMR (75.5MHz, CDCl₃): 200.0, 171.2, 144.7, 130.1, 130.1, 127.9, 126.9, 124.2, 82.6, 56.0, 55.7, 54.1, 42.6, 39.0, 38.6, 36.1, 35.9, 34.4, 33.2, 32.4, 25.8, 24.4, 22.0, 21.9, 21.2, 20.9, 17.8, 12.2

### Example 34: Synthesis of cis, trans-mixture of 4,17 (20)-pregnadien-3-one of the formula (I-E-4) from the compound of the formula (I-E-3) [Step Q]

The compound of the formula (I-E-3, -Ots, 35 g, 74 mmol) was dissolved in pyridine (100 mℓ) at the room temperature. The reaction mixture was heated to reflux at 120°C for 3 hours. After the completion of the reaction, pyridine solvent was removed under the reduced pressure. The resulted residue was dissolved in ethyl acetate (150 mℓ), and washed with 1 N HCl solution. The organic layer was dried over magnesium sulfate, filtered and evaporated under the reduced pressure to thereby yield the title compound (21.4 g, yield: 97%).
¹H-NMR (300MHz, CDCl₃): 5.74 (s, 1H), 5.14 (q, 1H), 2.44-0.8 (steroid main-body and 1.71 (d, 3H), 1.23 (s, 3H), 0.93 (s, 3H) = 28H)
¹³C NMR (75.5MHz, CDCl₃): 199.9, 171.7, 150.0, 124.21, 114.1, 56.0, 54.2, 44.5, 39.0, 37.2, 36.1, 35.5, 34.4, 33.3, 32.3, 31.7, 24.8, 21.6, 17.7, 17.2, 13.5

### Example 35: Synthesis of cis and trans isomer mixture of 4,17 (20)-pregnadien-3-one of the formula (I-E-4) from the compound of the formula. (I-E-3) [Step Q]

The compound of the formula (I-E-3, -Ots, 17.5 g, 37 mmol) was dissolved in absolute methanol (80 mℓ) at the room temperature. Sodium methoxide (6 g, 111 mmol) was added to the above solution, and the reaction mixture was heated to reflux for 4 hours. After the reaction was completed, the solvent was removed under the reduced pressure. The reaction mixture was dissolved in ethyl acetate (100 mℓ), and washed with brine. The organic layer was dried over magnesium sulfate, filtered and evaporated under the reduced pressure to thereby yield the title compound (10.1 g, yield: 91%).

### Example 36: Synthesis of the cis and trans isomer mixture of 4,17 (20)-pregnadiene-3-one of the formula (I-E-4) from the compound of the formula (I-E-3) [Step Q]

The compound of the formula (I-E-3, -Oms, 20 g, 51 mmol) was dissolved in pyridine (80 mℓ) at the room temperature. The reaction mixture was heated to reflux at 120°C for 2 hours. After the reaction was completed, the pyridine solvent was removed under the reduced pressure. The obtained reaction mixture was dissolved in ethyl acetate (100 mℓ) and washed with 1 N HCl solution. The organic layer dried over magnesium sulfate, filtered and evaporated under the reduced pressure to thereby yield the title compound (14.5 g, yield: 95%).

### Example 37: Synthesis of the cis and trans isomer mixture of 4,17 (20)-pregnadien-3-one of the formula (I-E-4) from the compound of the formula (I-E-3) [Step Q]

The compound of the formula (I-E-3, -Oms, 10 g, 25.5 mmol) was dissolved in absolute methanol (80 mℓ) at the room temperature. Sodium methoxide (5.5 g, 102 mmol) was added to the above solution and the reaction mixture was heated to reflux for 4 hours. After the reaction was completed, the solvent was removed under the reduced pressure. The resulted residue was dissolved in ethyl acetate (100 mℓ), and washed with brine. The organic layer dried over magnesium sulfate, filtered and evaporated under the reduced pressure to thereby yield the title compound (7.1 g, yield: 93%).

### Example 38: Synthesis of E-guggulsterone [4,17 (20)-E-pregnadiene-3,16-dione] of the formula (III) from the compound of the formula (II-A) [Step R]

*E-*guggulsterol (22 g,70 mmol) of the formula (II-A) was dissolved in dichloromethane (200 mℓ) at the room temperature. Activated manganese dioxide (MnO₂, 60 g, 700 mmol) was added to the above solution at once, followed by stirring vigorously for 2 hours. After the reaction was completed, excessive manganese dioxide was removed by filtration. The solvent was evaporated under the reduced pressure to thereby yield the title compound (20.6 g, yield: 94%).
¹H-NMR (300MHz, CDCl₃): 6.52 (q, 1H), 5.76 (s, 1H), 2.50-1.08 (steroid main-body and 1.89 (d, 3H), 1.25 (s, 3H), 1.08 (s, 3H) = 26H)
¹³C NMR (75.5MHz, CDCl₃): 206, 199.6, 170.5, 147.8, 129.9, 124.5, 53.8, 49.9, 43.5, 39.0, 38.2, 36.4, 35.9, 34.7, 34.3, 32.9, 32.2, 21.1, 17.9,17.7, 13.6

### Example 39: Synthesis of E-guggulsterone [4,17 (20)-E-pregnadiene-3,16-dione] of the formula (III) from the compound of the formula (II-A) [Step R]

*E*-guggulsterol (22 g, 70 mmol) of the formula (II-A) was dissolved in dichloromethane (300 mℓ) at the room temperature. Activated manganese dioxide (MnO₂, 120 g, 1.4 mol) was added to the above solution at once and stirred vigorously for 1 hour. After the reaction was completed, excessive manganese dioxide was removed by filtration. The solvent was evaporated under the reduced pressure to thereby yield the title compound (21.4 g, yield: 98%).

### Example 40: Synthesis of E-guggulsterone [4,17 (20)-E-pregnadiene-3,16-dione] of the formula (III) from the compound of the formula (II-B) [Step S]

The compound of the formula (II-B, 25g, 79mmol) was dissolved in benzene (200 mℓ) at the room temperature. Cyclohexanone (82 mℓ) and aluminum isopropoxide (8.1 g, 40 mmol) were added the above solution in order. The reaction mixture was heated up to reflux at 80°C for 2 hours. After the above mixture was cooled to the room temperature, 10% aqueous sulfuric acid (50 mℓ) was added, followed by stirring vigorously at the room temperature for 15 minutes. The benzene layer was separated and the remaining aqueous layer was extracted with ethyl acetate (150 mℓ). The combined organic layer was dried over magnesium sulfate, filtered and evaporated under the reduced pressure to thereby yield the title compound (22.8 g, yield: 92%, single *E*-guggulsterone).
¹H-NMR (300MHz, CDCl3): 6.52 (q, 1H), 5.76 (s, 1H), 2.50-1.08 (steroid main-body and 1.89 (d, 3H), 1.25 (s, 3H), 1.08 (s, 3H) = 26H)
¹³C- NMR (75.5MHz, CDCl3) : 206, 199.6, 170.5, 147.8, 129.9, 124.5, 53.8, 49.9, 43.5, 39.0, 38.2, 36.4, 35.9, 34.7, 34.3, 32.9, 32.2, 21.1, 17.9, 17.7, 13.6

### Example 41: Synthesis of E-guggulsterone [4,17 (20)-E-pregnadiene-3,16-dione] of the formula (III) from the compound of the formula (II-B) [Step S]

The compound of the formula (II-B, 25g, 79mmol) was dissolved in toluene (200 mℓ) at the room temperature. Cyclohexanone (82 mℓ) and aluminum isopropoxide (8.1 g, 40 mmol) were added the above solution in order. The reaction mixture was heated up to reflux at 120°C for 2 hours. After the above mixture was cooled to the room temperature, 10% aqueous sulfuric acid (50 mℓ) was added, followed by stirring vigorously at the room temperature for 15 minutes. The toluene layer was separated and the remaining aqueous layer was extracted with ethyl acetate (150 mℓ). The combined organic layer was dried over magnesium sulfate, filtered and evaporated under the reduced pressure to thereby yield the title compound (23.3 g, yield: 94%, *E*:*Z*=7:3 guggulsterone).

### Example 42: Synthesis of E-gugguisterone [4,17 (20)-E-pregnadiene-3,16-dione] of the formula (III) from the compound of the formula (II-B) [Step S]

The compound of the formula (II-B, 25g, 79mmol) was dissolved in toluene (200 mℓ) at the room temperature. Cyclohexanone (82 mℓ) and aluminum tri *sec*-butoxide (10g, 40 mmol) were added the above solution in order. The reaction mixture was heated up to reflux at 120 °C for 2 hours. After the above mixture was cooled to the room temperature, and 10% aqueous sulfuric acid (50 mℓ) was added, followed by stirring vigorously at the room temperature for 15 minutes. The toluene layer was separated and the remaining aqueous layer was extracted with ethyl acetate (150 mℓ). The combined organic layer was dried over magnesium sulfate, filtered and evaporated under the reduced pressure to thereby yield the title compound (22.5 g, yield: 91%, *E*:*Z*=3:1 guggulsterone).

### Example 43: Synthesis of E-guggulsterone [4,17 (20)-E-pregnadiene-3,16-dione] of the formula (III) from the compound of the formula (II-B) [Step S]

The compound of the formula (II-B, 8.3g, 26mmol) was dissolved in benzene (100 mℓ) at the room temperature. Methyl ethyl ketone (60 mℓ) and aluminum isopropoxide (3 g, 13 mmol) were added the above solution in turn. The reaction mixture was heated up to reflux at 80 °C for 4 hours. After the above mixture was cooled to the room temperature, and 10% aqueous sulfuric acid (15 mℓ) was added, followed by stirring vigorously at the room temperature for 15 minutes. The benzene layer was separated and the remaining aqueous layer was extracted with ethyl acetate (50 mℓ). The combined organic layer was dried over magnesium sulfate, filtered and evaporated under the reduced pressure to thereby yield the title compound (2.6 g, yield: 32%, single *E*-guggulsterone).

### Example 44: Synthesis of Z-guggulsterone [4,17 (20)-Z-pregnadiene-3,16-dione] of the formula (IV) from the compound of the formula (III) [Step T]

*E*-guggulsterone (1 g, 3.2 mmol) of the formula (III) was dissolved in dichloromethane (20 mℓ) at the room temperature and then methylene blue (5 mg) was dissolved therein. The reaction mixture was cooled to 0 °C and was irradiated by a 300 W tungsten bulb for 6 hours. The resultant mixture was passed through a flash silica column, and the solvent was removed by evaporation under the reduced pressure. The residue was chromatographed on silica eluting with ethyl acetate/hexane (v/v = 3/7) to thereby yield the title compound (433 mg, yield: 43.3%), and the unreacted *E*-guggulsterone (560 mg) was recovered.
¹H-NMR (300MHz, CDCl₃): 5.73 (m, 2H), 2.43-0.75 (steroid main-body and 2.08 (d, 3H), 1.22 (s, 3H), 0.96 (s, 3H) = 26H)
¹³C-NMR (75.5MHz, CDCl₃): 208.2, 199.6, 170.7, 148.2, 130.9, 124.5, 54.0, 49.4, 43.4, 39.7, 39.1, 35.9, 35.0, 34.3, 33.0, 32.2, 21.0, 19.9, 17.7, 14.5

### Example 45: Synthesis of Z-guggulsterone [4,17 (20)-Z-pregnadiene-3,16-dione] of the formula (IV) from the compound of the formula (III) [Step T]

*E*-guggulsterone (1 g, 3.2 mmol) of the formula (III) was dissolved in toluene (100 mℓ) at the room temperature. The reaction vessel was purged with nitrogen gas and blocked up entirely. The reaction was maintained at 160 °C for 2 hours, and was cooled to the room temperature. The solvent was removed under the reduced pressure. The resultant mixture was chromatographed on silica eluting with ethyl acetate/ hexane (v/v = 3/7) to thereby yield the title compound (586 mg, yield: 58.6%), and the unreacted *E-*guggulsterone (410 mg) was recovered.

### Example 46: Synthesis of Z-guggulsterone [4,17 (20)-Z-pregnadiene-3,16-dione] of the formula (IV) from the compound of the formula (III) [Step T]

*E-*guggulsterone (1 g, 3.2 mmol) of the formula (III) was dissolved in benzene (100 mℓ) with *p*-toluenesulfonic acid (100 mg) at the room temperature. The reaction mixture was heated up to reflux at 80 °C for 1 hour, and then cooled to the room temperature, followed by evaporation under the reduced pressure. The resultant mixture was chromatographed on silica eluting with ethyl acetate/ hexane (v/v = 3/7) to thereby yield the title compound (664 mg, yield: 64.4%), and the unreacted *E*-guggulsterone (350 mg) was recovered.

### INDUSTRIAL APPLICABILITY

According to the present invention, 4,17(20)-*E*-pregnadiene-3,16-dione (*E*-guggulsterone) of the formula (III) and an enantiomer thereof, 4,17 (20)-*Z*-pregnadiene-3,16-dione (*Z*-guggulsterone) of the formula (IV), having an effect of lowering the elevated low density lipoprotein (LDL) and high levels of the cholesterol effectively, and elevating the low levels of the high density lipoprotein (HDL), can be prepared in a high yield with low-cost.

## Claims

1. A process for the preparation of 4,17 (20)-*E*-pregnadiene-3,16-dione of the following formula (III), which comprises oxidizing the compound of the formula (II) to give 4,17 (20)-*E*-prcgnadiene-3,16-dione of the formula (III) 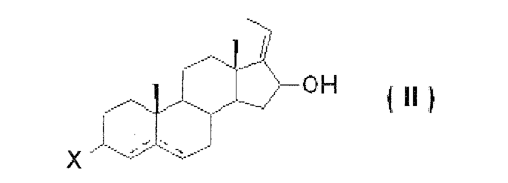 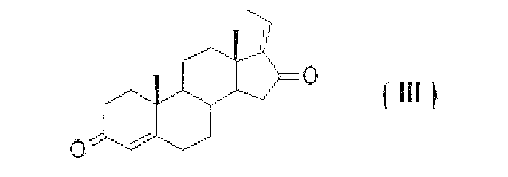 wherein X represents a hydroxyl (-OH) group or an oxo (=O) group, and a dotted line indicates the presence of a double bond at the position C-4 or C-5.

2. The process of claim 1, wherein the compound of formula (II) is prepared by protecting the compound of the formula (I-A) with 1,2-ethanedithiol to give the compound of the formula (I-A-1), subjecting the resulted compound of the formula (I-A-1) to the Wittig reaction to give the compound of the formula (I-A-2), eliminating the protection group to give the compound of the formula (I-A-3), and reacting the resulted compound with selenium dioxide (SeO₂).

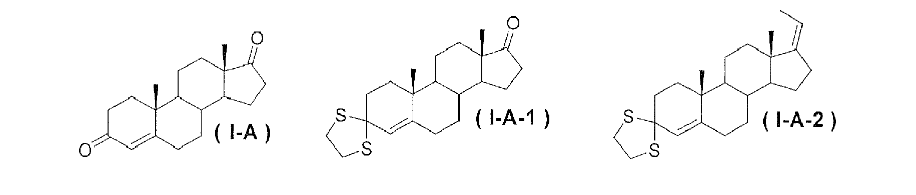

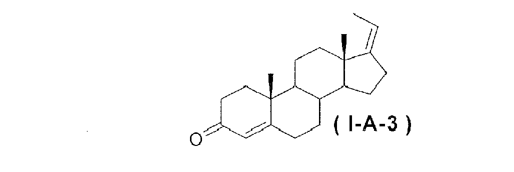

3. The process of claim 1, wherein the compound of formula (II) is prepared by subjecting the compound of the formula (I-B) to the Wittig reaction to give the compound of the formula (I-B-1), and reacting the compound of the formula (I-B-1) with selenium dioxide (SeO₂)

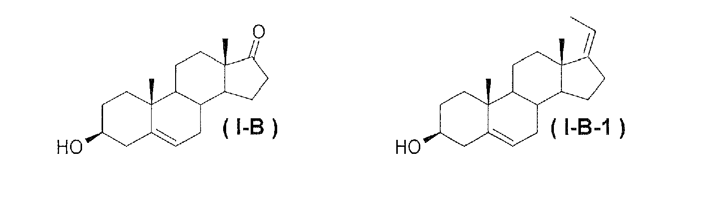

4. The process of claim 1, wherein the compound of formula (II) is prepared by subjecting the compound of the formula (I-B) to Wittig reaction to form the compound of the formula (I-B-1), oxidizing the resulted compound with pyridinium chlorochromate (PCC) to give the compound of the formula (I-B-2), reacting the resulted compound in the presence of a base to give the compound of the formula (I-B-3), and reacting the product with selenium dioxide.

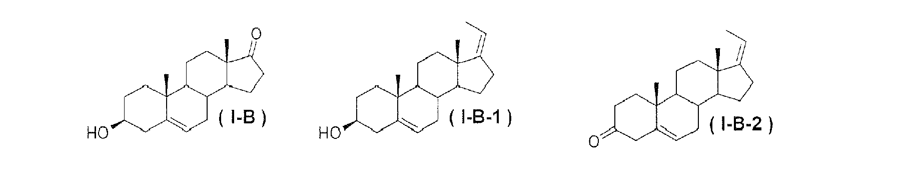

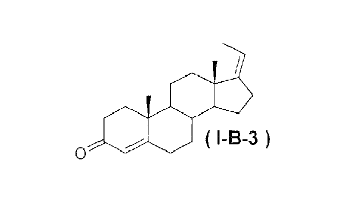

5. The process of claim 1, wherein the compound of formula (II) is prepared by protecting the compound of the formula (I-C) with 1,2-ethanediol in the presence of an acid catalyst to give the compound of the formula (I-C-1), subjecting the resulted compound to the Wittig reaction with ethyltriphenyl phosphonium halide in the presence of a strong base to give the compound of the formula (I-C-3), deprotecting the ketone group of the compound of the formula (I-C-3) to give the compound of the formula (I-A-3), and oxidizing the product with selenium dioxide.

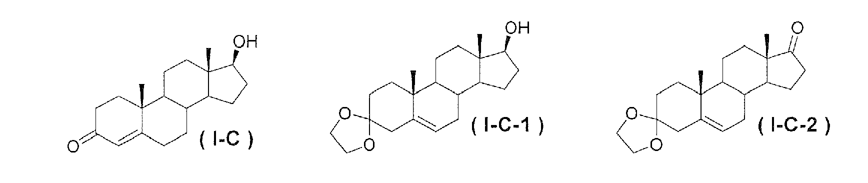

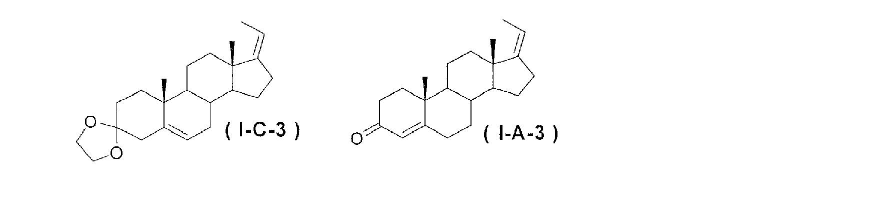

6. The process of claim 1, wherein the compound of formula (II) is prepared by reacting the compound of the formula (I-D) with hydrazine monohydrate in the presence of a base at 120 °C for 1 hour and then 160 °C for an additional 2 hours.

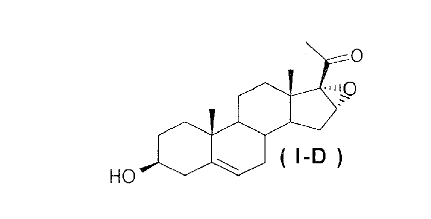

7. The process of claim 1, wherein the compound of formula (II) is prepared by reducing the compound of the formula (I-E) to give the compound of the formula (I-E-1), oxidizing the resulted compound by using an selective oxidizing reagent to give the compound of the formula (I-E-2), introducing a *p*-toluene sulfonyl group or methane sulfonyl group to the resulted compound of the formula (1-E-2) to give the compound of the formula (I-E-3), reacting the product with a base to give the compound of the formula (I-E-4), and reacting it with selenium dioxide.
